# EUROPEAN PATENT APPLICATION

(11) **EP 3 960 198 A1**
(43) Date of publication of application: **02.03.2022**
(21) Application number: 20795094.0
(22) Date of filing: 20.04.2020
(51) Int. Cl.: A61K 39/395, A61P 19/02, A61P 29/00, C07K 16/24, G01N 33/49, G01N 33/53

(54) **BIOMARKER FOR TREATING RHEUMATOID ARTHRITIS**

(30) Priority: 23.04.2019 JP 2019081452
(71) Applicant: Eisai R&D Management Co., Ltd., Tokyo 112-8088 (JP)
(72) Inventor: YASUDA, Nobuyuki, Kobe-shi, Hyogo 650-0047 (JP); YAMADA, Tomohiro, Kobe-shi, Hyogo 650-0047 (JP); TAGO, Fumitoshi, Tokyo 112-8088 (JP); HOJO, Seiichiro, Tokyo 112-8088 (JP); IMAI, Toshio, Kobe-shi, Hyogo 650-0047 (JP)
(74) Representative: Hoffmann Eitle
(86) International application number: PCT/JP2020/017016
(87) International publication number: WO 2020/218232

(57) **Abstract**

An object of the present invention is to provide a method for predicting a therapeutic effect of a drug that inhibits FKN-CX3CR1 interaction on rheumatoid arthritis in a rheumatoid arthritis subject, and novel and more effective therapeutic agent for rheumatoid arthritis exploiting the method. In order to predict a therapeutic effect of a drug that inhibits fractalkine (FKN)-CX3CR1 interaction in a rheumatoid arthritis subject, provided is a method comprising predicting the therapeutic effect of the drug in the subject on the basis of a measurement value of CD16+ monocytes in a biological sample obtained from the subject before the start of administration of the drug.

## Description

### [Technical Field]

The present invention relates to a method for predicting a therapeutic effect of a therapeutic agent for rheumatoid arthritis, specifically, a drug that inhibits fractalkine (FKN)-CX3CR1 interaction, in a rheumatoid arthritis subject, and a therapeutic agent for rheumatoid arthritis in a subject found to be likely to respond to the drug by the method.

### [Background Art]

Fractalkine (also referred to as "FKN") is a membrane-bound chemokine that is expressed on the surface of vascular endothelial cells by the inflammatory stimulus of LPS, TNF-α, IL-1, or the like. Cells expressing an FKN receptor CX3CR1 bind to membrane-bound FKN without the medium of selectin or integrin and cause strong cell adhesion. Secreted FKN shed from the membrane-bound FKN exhibits cell-migrating activity against NK cells, T cells, and monocytes having CX3CR1.

The expression of FKN is induced on the surface of vascular endothelial cells by proinflammatory cytokines. The increased expression of FKN and the accumulation of CX3CR1+ cytotoxic effector lymphocytes and macrophages have been reported as to patients having rheumatoid arthritis (also referred to as "RA").

Therapeutic effects brought about by the inhibition of FKN have previously been reported as to collagen-induced arthritis (CIA) murine models, which are known as chronic rheumatoid arthritis models (Non Patent Literature 1). The results obtained in CIA indicate significant reduction in the clinical score of arthritis, significant decrease in the incidence of arthritis, and significant decrease in inflammatory cells in synovial membranes and bony erosion, due to anti-fractalkine antibodies. Furthermore, it is suggested that an anti-fractalkine antibody inhibiting the interaction between FKN and CX3CR1 is capable of treating inflammatory diseases including rheumatoid arthritis (Patent Literature 1).

A plurality of mouse anti-human fractalkine (hFKN) monoclonal antibodies (clones 1F3-1, 3A5-2, 1F3, 1G1, 2B2, 3D5, 3H7, 6D1, 7F6, and 5H7-6) are disclosed as such antibodies. Particularly, the clone 3A5-2 has been humanized because of its high neutralizing activity, binding affinity, and interspecific cross reactivity against hFKN, and designated as H3-2L4 (Patent Literature 2, which is incorporated herein by reference in its entirety). Its effectiveness for human subjects with rheumatoid arthritis has been shown (Patent Literature 3, which is incorporated herein by reference in its entirety).

### [Citation List]

### [Non Patent Literature]

[Non Patent Literature 1] J Immunol. 2004; 173: 7010-7016

### [Patent Literature]

[Patent Literature 1] WO2006/046739
[Patent Literature 2] WO2011/052799
[Patent Literature 3] Japanese Patent Laid-Open No. 2017-193541

### [Summary of Invention]

### [Technical Problem]

As well known in the art, whether a subject is responsive even drugs known to be excellent in efficacy for certain diseases or not depends on the subject. Subjects having no response eventually bear only the risk of adverse reactions and economical burdens. Thus, a determination whether each subject is likely to respond to a certain drug or not prior to the treatment can make a determination easy whether to initiate administration of the drug or not and. This approach is very useful because therapeutic subjects unlikely to respond thereto can avoid adverse reactions or economical burdens beforehand.

An object of the present invention is to provide a method for predicting a therapeutic effect of a drug that inhibits FKN-CX3CR1 interaction on rheumatoid arthritis in a rheumatoid arthritis subject, and novel and more effective therapeutic agent for rheumatoid arthritis exploiting the method.

### [Solution to Problem]

The present invention encompasses the following embodiments.
(1) A method for predicting a therapeutic effect of a drug that inhibits fractalkine (FKN)-CX3CR1 interaction in a rheumatoid arthritis subject, the method comprising predicting the therapeutic effect of the drug in the subject on the basis of a measurement value of CD16+ monocytes in a biological sample obtained from the subject before the start of administration of the drug.
(2) The method according to (1), wherein
   the prediction comprises comparing the measurement value of CD16+ monocytes with a control.
(3) The method according to (2), wherein
   the measurement value of CD16+ monocytes equal to or higher than the control indicates that the subject is likely to respond to the drug.
(4) The method according to any of (1) to (3), wherein
   the biological sample is blood or synovial fluid.
(5) The method according to (4), wherein
   the biological sample is blood.
(6) The method according to any of (1) to (5), wherein
   the measurement value of CD16+ monocytes is a numerical value calculated from a ratio of the CD16+ monocytes to total monocytes.
(7) The method according to any of (1) to (6), wherein
   the drug is an anti-human FKN antibody or an antibody binding fragment thereof.
(8) The method according to (7), wherein
   the anti-human FKN antibody is a fully human, humanized or chimeric antibody.
(9) The method according to (7) or (8), wherein
   the anti-human FKN antibody comprises
   (a) CDR-H1 comprising the amino acid sequence of SEQ ID NO: 5 (NYYIH);
   (b) CDR-H2 comprising the amino acid sequence of SEQ ID NO: 6 (WIYPGDGSPKFNERFKG);
   (c) CDR-H3 comprising the amino acid sequence of SEQ ID NO: 7 (GPTDGDYFDY);
   (d) CDR-L1 comprising the amino acid sequence of SEQ ID NO: 8 (RASGNIHNFLA);
   (e) CDR-L2 comprising the amino acid sequence of SEQ ID NO: 9 (NEKTLAD); and
   (f) CDR-L3 comprising the amino acid sequence of SEQ ID NO: 10 (QQFWSTPYT).
(10) The method according to any of (7) to (9), wherein
   a heavy chain variable region of the anti-human FKN antibody comprises the amino acid sequence represented by SEQ ID NO: 3 
(QVQLVQSGAEVKKPGASVKVSCKASGYTFTNYYIHWVKQAPGQGLEWIGWIYPGDG
SPKFNERFKGRTTLTADKSTNTAYMLLSSLRSEDTAVYFCATGPTDGDYFDYWGQGT
TVTVSS) and
 and
   a light chain variable region thereof comprises the amino acid sequence represented by SEQ ID NO: 4 
(DIQMTQSPSSLSASVGDRVTITCRASGNIHNFLAWYQQKPGKAPKLLIYNEKTLAD

GVPSRFSGSGSGTDYTLTISSLQPEDFATYFCQQFWSTPYTFGGGTKVEIK).
(11) The method according to any of (7) to (10), wherein
   the anti-human FKN antibody comprises a constant region of human IgG2 isotype, and
   an Fc region of the constant region contains V234A and G237A mutations.
(12) The method according to any of (1) to (11), wherein
   the rheumatoid arthritis subject is a rheumatoid arthritis patient with an inadequate response to conventional treatment.
(13) A therapeutic agent for rheumatoid arthritis, comprising a drug that inhibits FKN-CX3CR1 interaction as an active ingredient, wherein
   the therapeutic agent for rheumatoid arthritis is used in a method for treating rheumatoid arthritis, comprising the steps of:
   identifying a subject predicted to be likely to respond to the drug on the basis of a measurement value of CD16+ monocytes in a biological sample obtained from a rheumatoid arthritis subject before the start of administration of the drug; and
   administering a therapeutically effective amount of the drug to the identified subject.
(14) The therapeutic agent for rheumatoid arthritis according to (13), wherein
   the step of identifying a subject comprises identifying a subject in which the measurement value of CD16+ monocytes is equal to or higher than a control.
(15) A therapeutic agent for rheumatoid arthritis, comprising a drug that inhibits FKN-CX3CR1 interaction as an active ingredient, wherein
   the therapeutic agent for rheumatoid arthritis is used in a method for treating rheumatoid arthritis, comprising the step of
   administering a therapeutically effective amount of the drug to a rheumatoid arthritis subject in which a measurement value of CD16+ monocytes in a biological sample obtained from the subject before the start of administration of the drug is equal to or higher than a control.
(16) The therapeutic agent for rheumatoid arthritis according to any of (13) to (15), wherein
   the biological sample is blood or synovial fluid.
(17) The therapeutic agent for rheumatoid arthritis according to (16), wherein
   the biological sample is blood.
(18) The therapeutic agent for rheumatoid arthritis according to any of (13) to (17), wherein
   the measurement value of CD16+ monocytes is a numerical value calculated from a ratio of the CD16+ monocytes to total monocytes.
(19) The therapeutic agent for rheumatoid arthritis according to any of (13) to (18), wherein
   the drug is an anti-human FKN antibody or an antibody binding fragment thereof.
(20) The therapeutic agent for rheumatoid arthritis according to (19), wherein
   the anti-human FKN antibody is a fully human, humanized or chimeric antibody.
(21) The therapeutic agent for rheumatoid arthritis according to (19) or (20), wherein
   the anti-human FKN antibody comprises
   (a) CDR-H1 comprising the amino acid sequence of SEQ ID NO: 5 (NYYIH);
   (b) CDR-H2 comprising the amino acid sequence of SEQ ID NO: 6 (WIYPGDGSPKFNERFKG);
   (c) CDR-H3 comprising the amino acid sequence of SEQ ID NO: 7 (GPTDGDYFDY);
   (d) CDR-L1 comprising the amino acid sequence of SEQ ID NO: 8 (RASGNIHNFLA);
   (e) CDR-L2 comprising the amino acid sequence of SEQ ID NO: 9 (NEKTLAD); and
   (f) CDR-L3 comprising the amino acid sequence of SEQ ID NO: 10 (QQFWSTPYT).
(22) The therapeutic agent for rheumatoid arthritis according to any of (19) to (21), wherein
   a heavy chain variable region of the anti-human FKN antibody comprises the amino acid sequence represented by SEQ ID NO: 3
      (QVQLVQSGAEVKKPGASVKVSCKASGYTFTNYYIHWVKQAPGQGLEWIGWIYPGDG SPKFNERFKGRTTLTADKSTNTAYMLLSSLRSEDTAVYFCATGPTDGDYFDYWGQGT TVTVSS) and
   a light chain variable region thereof comprises the amino acid sequence represented by SEQ ID NO: 4 
(DIQMTQSPSSLSASVGDRVTITCRASGNIHNFLAWYQQKPGKAPKLLIYNEKTLAD

GVPSRFSGSGSGTDYTLTISSLQPEDFATYFCQQFWSTPYTFGGGTKVEIK).
(23) The therapeutic agent for rheumatoid arthritis according to any of (19) to (22), wherein
   the anti-human FKN antibody comprises a constant region of human IgG2 isotype, and
   an Fc region of the constant region contains V234A and G237A mutations.
(24) The therapeutic agent for rheumatoid arthritis according to any of (13) to (23), wherein
   the rheumatoid arthritis subject is a rheumatoid arthritis patient with an inadequate response to conventional treatment.
(25) A method for treating rheumatoid arthritis, comprising the steps of:
   identifying a subject predicted to be likely to respond to a drug that inhibits FKN-CX3CR1 interaction on the basis of a measurement value of CD16+ monocytes in a biological sample obtained from a rheumatoid arthritis subject; and
   administering a therapeutically effective amount of the drug to the identified subject.
(26) The method for treating rheumatoid arthritis according to (25), wherein
   the step of identifying a subject comprises identifying a subject who is equal to or higher than a control.
(27) A method for treating rheumatoid arthritis, comprising the step of
   administering a therapeutically effective amount of a drug that inhibits FKN-CX3CR1 interaction to a rheumatoid arthritis subject in which a measurement value of CD16+ monocytes in a biological sample obtained from the subject before the start of administration of the drug is equal to or higher than a control.
(28) The method for treating rheumatoid arthritis according to any of (25) to (27), wherein
   the biological sample is blood or synovial fluid.
(29) The method for treating rheumatoid arthritis according to (28), wherein
   the biological sample is blood.
(30) The method for treating rheumatoid arthritis according to any of (25) to (29), wherein
   the measurement value of CD16+ monocytes is a numerical value calculated from a ratio of the CD16+ monocytes to total monocytes.
(31) The method for treating rheumatoid arthritis according to any of (25) to (30), wherein
   the drug is an anti-human FKN antibody or an antibody binding fragment thereof.
(32) The method for treating rheumatoid arthritis according to (31), wherein
   the anti-human FKN antibody is a fully human, humanized or chimeric antibody.
(33) The method for treating rheumatoid arthritis according to (31) or (32), wherein
   the anti-human FKN antibody comprises
   (a) CDR-H1 comprising the amino acid sequence of SEQ ID NO: 5 (NYYIH);
   (b) CDR-H2 comprising the amino acid sequence of SEQ ID NO: 6 (WIYPGDGSPKFNERFKG);
   (c) CDR-H3 comprising the amino acid sequence of SEQ ID NO: 7 (GPTDGDYFDY);
   (d) CDR-L1 comprising the amino acid sequence of SEQ ID NO: 8 (RASGNIHNFLA);
   (e) CDR-L2 comprising the amino acid sequence of SEQ ID NO: 9 (NEKTLAD); and
   (f) CDR-L3 comprising the amino acid sequence of SEQ ID NO: 10 (QQFWSTPYT).
(34) The method for treating rheumatoid arthritis according to any of (31) to (33), wherein
   a heavy chain variable region of the anti-human FKN antibody comprises the amino acid sequence represented by SEQ ID NO: 3
      (QVQLVQSGAEVKKPGASVKVSCKASGYTFTNYYIHWVKQAPGQGLEWIGWIYPGDG SPKFNERFKGRTTLTADKSTNTAYMLLSSLRSEDTAVYFCATGPTDGDYFDYWGQGT TVTVSS) and
   a light chain variable region thereof comprises the amino acid sequence represented by SEQ ID NO: 4 
(DIQMTQSPSSLSASVGDRVTITCRASGNIHNFLAWYQQKPGKAPKLLIYNEKTLAD

GVPSRFSGSGSGTDYTLTISSLQPEDFATYFCQQFWSTPYTFGGGTKVEIK).
(35) The method for treating rheumatoid arthritis according to any of (31) to (34), wherein
   the anti-human FKN antibody comprises a constant region of human IgG2 isotype, and
   an Fc region of the constant region contains V234A and G237A mutations.
(36) The method for treating rheumatoid arthritis according to any of (25) to (35), wherein
   the rheumatoid arthritis subject is a rheumatoid arthritis patient with an inadequate response to conventional treatment.

### [Advantageous Effects of Invention]

The present invention provides a method for predicting a therapeutic effect of a drug that inhibits FKN-CX3CR1 interaction on rheumatoid arthritis in a rheumatoid arthritis subject, and novel and more effective therapeutic agent for rheumatoid arthritis exploiting the method.

### [Brief Description of Drawings]

[Figure 1] Figure 1 shows the ACR response rate after 12 weeks of each dose group in a phase 1/2 clinical trial.
[Figure 2] Figure 2 shows the relationship between the ACR response and CD16+ monocyte ratio after 12 weeks of a 400 mg group in a phase 1/2 clinical trial. Figure 2A shows ACR20, Figure 2B shows ACR50, and Figure 2C shows ACR70. CD16+Mo denotes CD16+ monocytes, and Total Mo denotes total monocytes.
[Figure 3] Figure 3 shows the ACR response rate after 12 weeks of each dose group in a phase 2 clinical trial.
[Figure 4] Figure 4 shows the ACR response rate after 24 weeks of each dose group in a phase 2 clinical trial.
[Figure 5] Figure 5 shows the ACR response rate after 12 weeks of a population having less than the median value of a CD16+ monocyte ratio in a phase 2 clinical trial.
[Figure 6] Figure 6 shows the ACR response rate after 12 weeks of a population having the median value or more of a CD16+ monocyte ratio in a phase 2 clinical trial.
[Figure 7] Figure 7 shows the ACR response rate after 24 weeks of a population having less than the median value of a CD16+ monocyte ratio in a phase 2 clinical trial.
[Figure 8] Figure 8 shows the ACR response rate after 24 weeks of a population having the median value or more of a CD16+ monocyte ratio in a phase 2 clinical trial.

### [Description of Embodiments]

### 1. Summary of invention and definition

In one aspect, the present invention relates to a method for predicting a therapeutic effect of a drug that inhibits FKN-CX3CR1 interaction in a rheumatoid arthritis subject (hereinafter, also referred to as the "prediction method of the present invention").

In another aspect, the present invention also relates to a therapeutic agent for rheumatoid arthritis, comprising a drug that inhibits FKN-CX3CR1 interaction as an active ingredient (hereinafter, also referred to as the "therapeutic agent of the present invention").

In the present invention, the term "rheumatoid arthritis" refers to a disease state that can be diagnosed according to the 1987 ACR Classification Criteria or the 2010 ACR/EULAR Classification Criteria. Examples of physiological indexes of rheumatoid arthritis include symmetrical joint swelling and pain on passive movement, which are characteristics, but not invariable, of rheumatoid arthritis.

In the present invention, the rheumatoid arthritis subject can be a human or a non-human mammal (monkey, mouse, rat, rabbit, cattle, horse, goat, etc.). In the present invention, the rheumatoid arthritis subject is preferably a human.

In one embodiment of the present invention, the term "response" or "respond" can mean that therapeutically effective amelioration is exhibited for one or more items or parameters prescribed in evaluation criteria for rheumatoid arthritis established in the art. Examples of such items or parameters can include, but are not limited to, ACR (American College of Rheumatology) 20 response rates, ACR50 response rates, ACR70 response rates, erythrocyte sedimentation rates (ESR), high sensitive C-reactive protein (hs-CRP), HAQ (health assessment questionnaire), SDAI (simple disease activity index; e.g., disease activity score using CRP (DAS28-CRP)), CDAI (clinical disease activity index), and Boolean remission rates.

In one embodiment of the present invention, the time of evaluation of therapeutically effective amelioration is 1 week, 2 weeks, 3 weeks, 4 weeks, 5 weeks, 6 weeks, 7 weeks, 8 weeks, 9 weeks, 10 weeks, 12 weeks, 16 weeks, 20 weeks, 24 weeks, 28 weeks, 32 weeks, 36 weeks, 40 weeks, 44 weeks, 48 weeks, 52 weeks, 58 weeks, 60 weeks, 64 weeks, 68 weeks, 72 weeks, 76 weeks, 80 weeks, 80 weeks, 88 weeks, 92 weeks, 100 weeks, 104 weeks, 108 weeks, 112 weeks or 116 weeks after the start of treatment. In another embodiment, the time of evaluation of therapeutically effective amelioration is 4 weeks, 8 weeks, 12 weeks, 16 weeks or 24 weeks after the start of treatment. In an alternative embodiment, the time of evaluation of therapeutically effective amelioration is 12 weeks or 24 weeks. In a further embodiment, the time of evaluation of therapeutically effective amelioration is 24 weeks.

In one embodiment of the present invention, the therapeutically effective amelioration in rheumatoid arthritis can mean an ACR20 response rate of at least 10%, at least 20%, at least 30%, at least 40%, at least 50%, at least 60%, at least 70%, at least 80%, or at least 90%. In another embodiment, the ACR20 response rate is an ACR20 response rate at 1 week, 2 weeks, 3 weeks, 4 weeks, 5 weeks, 6 weeks, 7 weeks, 8 weeks, 9 weeks, 10 weeks, 12 weeks, 16 weeks, 20 weeks, 24 weeks, 28 weeks, 32 weeks, 36 weeks, 40 weeks, 44 weeks, 48 weeks, 52 weeks, 58 weeks, 60 weeks, 64 weeks, 68 weeks, 72 weeks, 76 weeks, 80 weeks, 80 weeks, 88 weeks, 92 weeks, 100 weeks, 104 weeks, 108 weeks, 112 weeks or 116 weeks after the start of treatment. In an alternative embodiment, the ACR20 response rate is an ACR20 response rate at 4 weeks, 8 weeks, 12 weeks, 16 weeks or 24 weeks after the start of treatment. In an alternative embodiment, the ACR20 response rate is an ACR20 response rate at 12 weeks or 24 weeks after the start of treatment. In a further embodiment, the ACR20 response rate is an ACR20 response rate at 24 weeks after the start of treatment.

In one embodiment of the present invention, the therapeutically effective amelioration in rheumatoid arthritis can mean an ACR50 response rate of at least 10%, at least 20%, at least 30%, at least 40%, at least 50%, at least 60%, at least 70%, at least 80%, or at least 90%. In another embodiment, the ACR50 response rate is an ACR50 response rate at 1 week, 2 weeks, 3 weeks, 4 weeks, 5 weeks, 6 weeks, 7 weeks, 8 weeks, 9 weeks, 10 weeks, 12 weeks, 16 weeks, 20 weeks, 24 weeks, 28 weeks, 32 weeks, 36 weeks, 40 weeks, 44 weeks, 48 weeks, 52 weeks, 58 weeks, 60 weeks, 64 weeks, 68 weeks, 72 weeks, 76 weeks, 80 weeks, 80 weeks, 88 weeks, 92 weeks, 100 weeks, 104 weeks, 108 weeks, 112 weeks or 116 weeks after the start of treatment. In an alternative embodiment, the ACR20 response rate is an ACR50 response rate at 4 weeks, 8 weeks, 12 weeks, 16 weeks or 24 weeks after the start of treatment. In an alternative embodiment, the ACR50 response rate is an ACR50 response rate at 12 weeks or 24 weeks after the start of treatment. In a further embodiment, the ACR50 response rate is an ACR50 response rate at 24 weeks after the start of treatment.

In one embodiment of the present invention, the therapeutically effective amelioration in rheumatoid arthritis can mean an ACR70 response rate of at least 10%, at least 20%, at least 30%, at least 40%, at least 50%, at least 60%, at least 70%, at least 80%, or at least 90%. In another embodiment, the ACR70 response rate is an ACR70 response rate at 1 week, 2 weeks, 3 weeks, 4 weeks, 5 weeks, 6 weeks, 7 weeks, 8 weeks, 9 weeks, 10 weeks, 12 weeks, 16 weeks, 20 weeks, 24 weeks, 28 weeks, 32 weeks, 36 weeks, 40 weeks, 44 weeks, 48 weeks, 52 weeks, 58 weeks, 60 weeks, 64 weeks, 68 weeks, 72 weeks, 76 weeks, 80 weeks, 80 weeks, 88 weeks, 92 weeks, 100 weeks, 104 weeks, 108 weeks, 112 weeks or 116 weeks after the start of treatment. In an alternative embodiment, the ACR20 response rate is an ACR70 response rate at 4 weeks, 8 weeks, 12 weeks, 16 weeks or 24 weeks after the start of treatment. In an alternative embodiment, the ACR70 response rate is an ACR70 response rate at 12 weeks or 24 weeks after the start of treatment. In a further embodiment, the ACR70 response rate is an ACR70 response rate at 24 weeks after the start of treatment.

In one embodiment, the rheumatoid arthritis subject according to the present invention is a human subject with inadequate response to, with no sustained response to, or with intolerance to an conventional therapeutic drug for rheumatoid arthritis. Examples of such an conventional therapeutic drug for rheumatoid arthritis include, but are not limited to, methotrexate, salazosulfapyridine, bucillamine, iguratimod and anti-TNF drugs (adalimumab, infliximab, golimumab, certolizumab pegol and etanercept).

Monocytes are known to include at least three subsets: CD14⁺⁺CD16⁻ (classical monocytes), CD14⁺⁺CD16⁺ (intermediate monocytes) and CD14⁺CD16⁺⁺ (non-classical monocytes), on the basis of their surface markers.

In the present invention, the "CD16+ monocytes" refer to any monocyte expressing CD16, i.e., both CD14⁺⁺CD16⁺ and CD14⁺CD16⁺⁺ monocytes. In this respect, the "measurement value of CD16+ monocytes" used in the present specification refers to the total amount of CD14⁺⁺CD16⁺ and CD14⁺CD16⁺⁺ monocytes measured in a given biological sample.

In the present invention, examples of the drug that inhibits FKN-CX3CR1 interaction include, but are not limited to, arbitrary compounds that inhibit the interaction between FKN and CX3CR1, such as antibodies that inhibit the interaction between FKN and CX3CR1, nucleic acids encoding the antibodies, antagonists of CX3CR1, partial agonists of CX3CR1, and inverse agonists of CX3CR1.

### 2. Method for predicting therapeutic effect of drug

The prediction method of the present invention comprises predicting the therapeutic effect of a drug that inhibits FKN-CX3CR1 interaction in a rheumatoid arthritis subject on the basis of a measurement value of CD16+ monocytes in a biological sample obtained from the subject before the start of administration of the drug.

In the present invention, the biological sample obtained from the rheumatoid arthritis subject is not particularly limited as long as monocytes can be collected therefrom. The biological sample can be, for example, a tissue or a body fluid. Examples of such a biological sample include, but are not limited to, blood such as whole blood, peripheral blood mononuclear cells (PMBC), and red blood cell-depleted whole blood, synovial fluid, and bone marrow fluid.

In one embodiment of the present invention, blood or synovial fluid is used as the biological sample. Blood is preferably used as the biological sample for reasons such as convenient obtainment and treatment and the detection sensitivity of CD16+ monocytes.

The biological sample obtained from the subject may be the biological sample itself collected from the subject, or the collected biological sample may be subjected to a treatment, such as dilution, concentration, or separation, which is usually performed. Examples of the sample thus treated include peripheral blood mononuclear cells (PMBC), red blood cell-depleted whole blood, and mononuclear cells isolated from synovial fluid by a density gradient centrifugation method.

The collection of the biological sample from the rheumatoid arthritis subject used in the present invention, the treatment of the collected biological sample, and the measurement of CD16+ monocytes can be performed by a physician or a person who has taken physician's instruction (who is not limited to a physician).

The biological sample derived from the rheumatoid arthritis subject used in the present invention may be obtained upon implementation of the present invention, or may be obtained or treated and stored in advance.

A standard approach capable of quantitatively measuring CD16+ monocytes can be used in the measurement of CD16+ monocytes in the obtained biological sample. Examples of such an approach can include, but are not limited to, flow cytometry (e.g., fluorescence activated cell sorting (FACS)).

In one embodiment of the present invention, the ratio of CD16+ monocytes is used as the measurement value of CD16+ monocytes. Examples of the ratio of CD16+ monocytes include the ratio of the CD16+ monocytes to total monocytes, the ratio of the CD16+ monocytes to total white blood cells, and the ratio of the CD16+ monocytes to total mononuclear cells. Flow cytometry is preferably used in the measurement of total monocytes, total white blood cells, total mononuclear cells and the CD16+ monocytes. In this embodiment, total monocytes, total white blood cells or total mononuclear cells are measured on the basis of forward scattering light (FSC) and side scattering light (SSC) according to a standard approach. Subsequently, monocytes are developed with CD14 and CD16, and CD16+ monocytes among the monocytes are measured. Subsequently, the ratio of the CD16+ monocytes is calculated on the basis of their numerical values. A commercially available anti-CD14 antibody and anti-CD16 antibody can be used in the identification of CD14 and CD16. If necessary, antibodies labeled with a fluorescence label or the like can be used. One example of a specific approach of calculating the ratio of the CD16+ monocytes to total monocytes by use of flow cytometry will be illustrated in Examples given below.

In the prediction method of the present invention, the prediction of the therapeutic effect based on the measurement value of CD16+ monocytes is performed by evaluating the measurement value. The evaluation can be performed, for example, by comparison with a control such as a standard value preset in order to discriminate between a response and a non-response (cutoff value), or a measurement value obtained in a subject who responded to the drug and/or a subject who did not respond to the drug. Methods for obtaining the measurement value of CD16+ monocytes to be evaluated and the control measurement value of CD16+ monocytes are preferably the same. The evaluation of the measurement value may be performed subsequently and continuously to the obtainment of the measurement value, or may be performed later using the measurement value obtained beforehand.

The control can be set on the basis of measurement values of CD16+ monocytes in one or more (e.g., 2, 3, 4, 5, 6, 7, 8, 9, 10, 15, 20, 25, 30, 35 or 40 individuals, or more) subjects who responded and/or did not respond to the drug.

The standard value can be set, for example, by subjecting measurement values of CD16+ monocytes obtained from a subject group found to have a response to the drug that inhibits FKN-CX3CR1 interaction to be evaluated, and measurement values of CD16+ monocytes obtained from a subject group found to have no response thereto, to ROC analysis (receiver operating characteristic analysis) or the like. The ROC analysis is, for example, an analysis method capable of evaluating the detection performance or diagnostic performance of a method for testing a disease, and is described in, for example, Japanese Journal of Clinical Laboratory Automation "Evaluation Manual for Diagnostic Usefulness of Clinical Test" Ver. 1.3 (2004.9.1), Vol. 29 Suppl. 1 (Vol. 154) (issued on September 1, 2004).

The standard value may be a most distinct measurement value of CD16+ monocytes for each group set by analyzing measurement values of CD16+ monocytes obtained from a subject group found to have a response to the drug that inhibits FKN-CX3CR1 interaction to be evaluated, and measurement values of CD16+ monocytes obtained from a subject group found to have no response thereto, using a histogram on a subject group basis.

The standard value may be a mean ± standard deviation or a quantile value of measurement values of CD16+ monocytes obtained from subjects found to have a response to the drug that inhibits FKN-CX3CR1 interaction to be evaluated, a mean ± standard deviation or a quantile value of measurement values of CD16+ monocytes obtained from subjects found to have no response to the drug that inhibits FKN-CX3CR1 interaction, or a mean ± standard deviation or a quantile value of measurement values of CD16+ monocytes obtained from rheumatoid arthritis subjects. Examples of the quantile value include median values, first tertile values, second tertile values, first quartile values, and third quartile values.

The standard value may be each individually set for a treatment method or a therapeutic drug, and the background of a subject (group). Improvement in the standard value may be determined according to methods for measuring and calculating the measurement value of CD16+ monocytes used, a statistical approach used, the type of a sample, the number of samples, etc. Thus, the established standard value may be adjusted so as to be larger or smaller on the basis of regular reevaluation, a treatment method or a therapeutic drug, or change in the distribution of population.

In another embodiment, the standard value may be a measurement value of CD16+ monocytes preset on the basis of a simulation model.

The subjects found to have a response to the drug that inhibits FKN-CX3CR1 interaction to be evaluated may be further subdivided into a subject who had a slight response to the drug that inhibits FKN-CX3CR1 interaction to be evaluated, a subject who had a moderate response thereto, and a subject who had a marked response thereto, etc., and their respective standard values m be set.

For the evaluation, only one member selected from the controls listed above may be used, or a plurality thereof may be used.

In one embodiment of the present invention, when the measurement value of CD16+ monocytes measured in a biological sample obtained from a rheumatoid arthritis subject before the start of administration is equal to or higher than the standard value set from subjects found to have a response to the drug that inhibits FKN-CX3CR1 interaction to be evaluated, the subject is evaluated as being likely to respond to the drug.

In another embodiment of the present invention, when the measurement value of CD16+ monocytes measured in a biological sample obtained from a rheumatoid arthritis subject before the start of administration is equal to or higher than the standard value set from subjects found to have no response to the drug that inhibits FKN-CX3CR1 interaction to be evaluated, the subject is evaluated as being likely to respond to the drug.

In a further alternative embodiment of the present invention, when the measurement value of CD16+ monocytes measured in a biological sample obtained from a rheumatoid arthritis subject before the start of administration is equal to or more than the cutoff value set from rheumatoid arthritis subjects, the subject is evaluated as being likely to respond to the drug.

In a further alternative embodiment of the present invention, when the measurement value of CD16+ monocytes measured in a biological sample obtained from a rheumatoid arthritis subject before the start of administration is two or more, or three or more standard values selected from a standard value set from subjects found to have a response to the drug that inhibits FKN-CX3CR1 interaction to be evaluated, a cutoff value set from subjects found to have no response to the drug that inhibits FKN-CX3CR1 interaction to be evaluated, and a cutoff value set from rheumatoid arthritis subjects, the subject is evaluated as being likely to respond to the drug.

In a certain embodiment of the present invention, the evaluation of a response is performed on the basis of ACR20, ACR50 or ACR70.

When the measurement value of CD16+ monocytes is the ratio of the CD16+ monocytes to total monocytes, the cutoff value can be set within the range of, for example, 6% to 16%. The range of the measurement value within which the subject is evaluated as being likely to respond to the drug that inhibits FKN-CX3CR1 interaction is, for example, 6.0% or more, 6.5% or more, 7.0% or more, 7.5% or more, 8.0% or more, 8.5% or more, 9.0% or more, 9.5% or more, 10.0% or more, 10.5% or more, 11.0% or more, 11.5% or more, 12.0% or more, 12.5% or more, 13.0% or more, 13.5% or more, 14.0% or more, 14.5% or more, 15.0% or more, 15.5% or more, and 16.0% or more.

The present prediction methods enable a therapy of rheumatoid arthritis by administering a therapeutically effective amount of the drug that inhibits FKN-CX3CR1 interaction in a rheumatoid arthritis subject evaluated as being likely to respond to the drug.

### 3. Therapeutic agent for rheumatoid arthritis

In one aspect, the present invention relates to a therapeutic agent for rheumatoid arthritis, comprising a drug that inhibits FKN-CX3CR1 interaction as an active ingredient.

In one embodiment, the therapeutic agent of the present invention is used in a method for treating rheumatoid arthritis, comprising the steps of: identifying a subject predicted to be likely to respond to the drug that inhibits FKN-CX3CR1 interaction on the basis of a measurement value of CD16+ monocytes in a biological sample obtained from a rheumatoid arthritis subject before the start of administration of the drug; and administering a therapeutically effective amount of the drug to the identified subject.

In another embodiment, the therapeutic agent of the present invention is used in a method for treating rheumatoid arthritis, a method for treating rheumatoid arthritis, comprising the step of administering a therapeutically effective amount of the drug that inhibits FKN-CX3CR1 interaction to a rheumatoid arthritis subject in which a measurement value of CD16+ monocytes in a biological sample obtained from the subject before the start of administration of the drug is equal to or higher than a control.

The measurement of CD16+ monocytes in a biological sample, the prediction or the comparison used in the method for treating rheumatoid arthritis can be performed in accordance with the prediction method of the present invention which described above.

### 4. Drug that inhibits FKN-CX3CR1 interaction

Examples of the drug that inhibits FKN-CX3CR1 interaction according to the present invention include, but are not limited to, arbitrary compounds that inhibit the interaction between FKN and CX3CR1, such as antibodies that inhibit the interaction between FKN and CX3CR1, nucleic acids encoding the antibodies, antagonists of CX3CR1, partial agonists of CX3CR1, and inverse agonists of CX3CR1. The antibody that inhibits the interaction between FKN and CX3CR1 is, for example, an anti-FKN antibody or an anti-CX3CR1 antibody. The drug that inhibits the interaction between FKN and CX3CR1 can be obtained by use of a known method for screening for an FKN-CX3CR1 inhibitor, for example, a screening method described in Japanese Patent Laid-Open No. 2002-345454.

In a preferred embodiment, the drug that inhibits FKN-CX3CR1 interaction is an anti-fractalkine (FKN) antibody. In the present invention, the anti-FKN antibody can be a fully human, humanized or chimeric antibody.

In the most preferred embodiment, the drug that inhibits FKN-CX3CR1 interaction is humanize d anti-human fractalkine antibody H3-2L4, or an antibody functionally equivalent thereto. In the present invention, the "functionally equivalent antibody" refers to an antibody that is equivalent to the antibody H3-2L4 in terms of at least any of or preferably all of binding affinity, neutralizing activity, cross reactivity, and pharmacokinetics in blood against human FKN. The functionally equivalent antibody also includes antibodies prescribed according to requirements for active ingredients in GUIDELINES ON EVALUATION OF SIMILAR BIOTHERAPEUTIC PRODUCTS (SBPs).

In the present invention, the term "anti-FKN antibody" may include an antigen binding fragment thereof. Such an antigen binding fragment is not particularly limited as long as the antigen binding fragment is a functional and structural fragment of the anti-FKN antibody, maintains the binding activity of the antibody against FKN, and does not significantly differ in pharmacokinetics in blood from the complete antibody. Examples of the antigen binding fragment of the antibody include, but are not limited to, Fab, Fab', F(ab')₂, Fv, single chain (scFv), their mutants, fusion proteins containing the antibody moiety, and other modified structures of immunoglobulin molecules containing an antigen recognition site.

In one embodiment, the anti-FKN antibody of the present invention can be an arbitrary antibody comprising the following CDR sequences:
(a) CDR-H1 comprising the amino acid sequence of SEQ ID NO: 5 (NYYIH);
(b) CDR-H2 comprising the amino acid sequence of SEQ ID NO: 6 (WIYPGDGSPKFNERFKG);
(c) CDR-H3 comprising the amino acid sequence of SEQ ID NO: 7 (GPTDGDYFDY);
(d) CDR-L1 comprising the amino acid sequence of SEQ ID NO: 8 (RASGNIHNFLA);
(e) CDR-L2 comprising the amino acid sequence of SEQ ID NO: 9 (NEKTLAD); and
(f) CDR-L3 comprising the amino acid sequence of SEQ ID NO: 10 (QQFWSTPYT).

In another embodiment, the anti-FKN antibody can be an antibody comprising a heavy chain and a light chain, wherein a heavy chain variable region of the antibody comprises the amino acid sequence of SEQ ID NO: 11
(QVQLVQSGAEVKKPGASVKVSCKASGYTFTNYYIHWVKQAPGQGLEWIGWIYPGDG SPKFNERFKGRTTLTADKSTNTAYMLLSSLRSDDTAVYFCATGPTDGDYFDYWGQGT TVTVSS), SEQ ID NO: 3
(QVQLVQSGAEVKKPGASVKVSCKASGYTFTNYYIHWVKQAPGQGLEWIGWIYPGDG SPKFNERFKGRTTLTADKSTNTAYMLLSSLRSEDTAVYFCATGPTDGDYFDYWGQGT TVTVSS), SEQ ID NO: 12
(QVQLVQSGAEVKKPGASVKVSCKASGYTFTNYYIHWVRQAPGQGLEWIGWIYPGDG SPKFNERFKGRTTLTRDKSTNTAYMELSSLRSDDTAVYFCATGPTDGDYFDYWGQGT TVTVSS), SEQ ID NO: 13
(QVQLVQSGAEVKKPGASVKVSCKASGYTFTNYYIHWVRQAPGQGLEWIGWIYPGDG SPKFNERFKGRTTMTADTSTSTAYMELSSLRSEDTAVYFCARGPTDGDYFDYWGQGT TVTVSS), or SEQ ID NO: 14
(QVQLVQSGAEVKKPGASVKVSCKASGYTFTNYYIHWVRQAPGQGLEWIGWIYPGDG SPKFNERFKGRTTLTADKSTSTAYMELSSLRSEDTAVYFCARGPTDGDYFDYWGQGT TVTVSS) and a light chain variable region of the antibody comprises the amino acid sequence of SEQ ID NO: 15
(DIQMTQSPSSLSASVGDRVTITCRASGNIHNFLAWYQQKPGKAPKFLVYNEKTLAD GVPSRFSGSGSGTQYTLTISSLQPEDFATYFCQQFWSTPYTFGGGTKVEIK), SEQ ID NO: 4
(DIQMTQSPSSLSASVGDRVTITCRASGNIHNFLAWYQQKPGKAPKLLIYNEKTLAD GVPSRFSGSGSGTDYTLTISSLQPEDFATYFCQQFWSTPYTFGGGTKVEIK), or SEQ ID NO: 16

(DIQMTQSPSSLSASVGDRVTITCRASGNIHNFLAWYQQKPGKAPKLLIYNEKTLAD
GVPSRFSGSGSGTQYTLTISSLQPEDFATYFCQQFWSTPYTFGGGTKVEIK).

In a preferred embodiment, the anti-FKN antibody can be an antibody comprising a heavy chain and a light chain, wherein a heavy chain variable region of the antibody comprises the amino acid sequence of SEQ ID NO: 3 
(QVQLVQSGAEVKKPGASVKVSCKASGYTFTNYYIHWVKQAPGQGLEWIGWIYPGDG
SPKFNERFKGRTTLTADKSTNTAYMLLSSLRSEDTAVYFCATGPTDGDYFDYWGQGT
TVTVSS)
 and a light chain variable region of the antibody comprises the amino acid sequence of SEQ ID NO: 4

(DIQMTQSPSSLSASVGDRVTITCRASGNIHNFLAWYQQKPGKAPKLLIYNEKTLAD

GVPSRFSGSGSGTDYTLTISSLQPEDFATYFCQQFWSTPYTFGGGTKVEIK).

In a particular embodiment, the anti-FKN antibody is an antibody comprising a constant region of human IgG2 isotype.

In a particular embodiment, the anti-FKN antibody is an antibody wherein a Fc region of the constant region of human IgG2 isotype contains V234A and/or G237A mutations.

In a particularly preferred embodiment of the present invention, the anti-FKN antibody is antibody H3-2L4 consisting of a heavy chain consisting of the amino acid sequence represented by SEQ ID NO: 1
(QVQLVQSGAEVKKPGASVKVSCKASGYTFTNYYIHWVKQAPGQGLEWIGWIYPGDG SPKFNERFKGRTTLTADKSTNTAYMLLSSLRSEDTAVYFCATGPTDGDYFDYWGQGT TVTVSSASTKGPSVFPLAPCSRSTSESTAALGCLVKDYFPEPVTVSWNSGALTSGVH TFPAVLQSSGLYSLSSVVTVPSSNFGTQTYTCNVDHKPSNTKVDKTVERKCCVECPP CPAPPAAAPSVFLFPPKPKDTLMISRTPEVTCVVVDVSHEDPEVQFNWYVDGVEVHN AKTKPREEQFNSTFRVVSVLTVVHQDWLNGKEYKCKVSNKGLPAPIEKTISKTKGQP REPQVYTLPPSREEMTKNQVSLTCLVKGFYPSDIAVEWESNGQPENNYKTTPPMLDS DGSFFLYSKLTVDKSRWQQGNVFSCSVMHEALHNHYTQKSLSLSPGK) and a light chain consisting of the amino acid sequence represented by SEQ ID NO: 2

(DIQMTQSPSSLSASVGDRVTITCRASGNIHNFLAWYQQKPGKAPKLLIYNEKTLAD
GVPSRFSGSGSGTDYTLTISSLQPEDFATYFCQQFWSTPYTFGGGTKVEIKRTVAAP
SVFIFPPSDEQLKSGTASVVCLLNNFYPREAKVQWKVDNALQSGNSQESVTEQDSKD
STYSLSSTLTLSKADYEKHKVYACEVTHQGLSSPVTKSFNRGEC).

Needless to say, an antibody derived from any of the anti-FKN antibodies listed above by appropriate alteration (e.g., modification of the antibody or partial substitution, addition, or deletion of the amino acid sequence of the antibody) so as to maintain the functions of the antibody or in order to add or improve the functions of the antibody is also included in the antibody of the present invention. More specifically, an antibody lacking lysine (Lys) positioned at the carboxy terminus (C terminus) of the heavy chain by an artificial method such as genetic engineering in order to decrease heterogeneity among antibodies produced by antibody-producing cells is also included in the scope of the present invention. In addition, the anti-FKN antibody contained in the therapeutic agent for rheumatoid arthritis of the present invention is not necessarily required to have complete homogeneity. For example, an anti-FKN antibody lacking lysine (Lys) positioned at the carboxy terminus (C terminus) of the heavy chain and an anti-FKN antibody that does not lack this lysine may coexist in the anti-FKN antibody as long as the intended functions of the therapeutic agent for rheumatoid arthritis of the present invention is maintained.

The anti-FKN antibody may be modified, if desired. The modification of the anti-FKN antibody may be a modification that changes (a) the three-dimensional structure of an amino acid sequence in a modification region, such as sheet or helix conformation; (b) the electric charge or hydrophobic status of the molecule at a target site; or (c) the effects of a modification on the maintenance of side chain volume, or may be a modification by which these changes are not clearly observed.

The modification of the anti-FKN antibody may be achieved by, for example, the substitution, deletion, and/or addition of a constituent amino acid residue(s).

In the present specification, the amino acid is used in the broadest sense thereof and includes not only natural amino acids, for example, serine (Ser), asparagine (Asn), valine (Val), leucine (Leu), isoleucine (Ile), alanine (Ala), tyrosine (Tyr), glycine (Gly), lysine (Lys), arginine (Arg), histidine (His), aspartic acid (Asp), glutamic acid (Glu), glutamine (Gln), threonine (Thr), cysteine (Cys), methionine (Met), phenylalanine (Phe), tryptophan (Trp), and proline (Pro) but non-natural amino acids such as amino acid mutants and derivatives. Those skilled in the art should understand, by taking this wide definition into consideration, that examples of the amino acid in the present specification include: L-amino acids; D-amino acids; chemically modified amino acids such as amino acid mutants and amino acid derivatives; amino acids, such as norleucine, β-alanine, and ornithine, which do not serve as materials constituting proteins *in vivo*; and chemically synthesized compounds having the characteristics of amino acids generally known to those skilled in the art. Examples of the non-natural amino acids include α-methylamino acids (α-methylalanine, etc.), D-amino acids (D-aspartic acid, D-glutamic acid, etc.), histidine-like amino acids (2-amino-histidine, β-hydroxy-histidine, homohistidine, α-fluoromethyl-histidine, α-methyl-histidine, etc.), amino acids having extra methylene in their side chains ("homo" amino acids), and amino acids in which a carboxylic acid functional group in the side chain is replaced with a sulfonic acid group (cysteic acid, etc.).

Naturally occurring amino acid residues can be classified into, for example, the following groups based on general side chain characteristics:
(1) hydrophobic residues: Met, Ala, Val, Leu, and Ile;
(2) neutral hydrophilic residues: Cys, Ser, and Thr;
(3) acidic residues: Asp and Glu;
(4) basic residues: Asn, Gln, His, Lys, and Arg;
(5) residues influencing chain orientation: Gly and Pro; and
(6) aromatic residues: Trp, Tyr, and Phe.

The non-conservative substitution of an amino acid sequence constituting the anti-FKN antibody may be performed by replacing an amino acid belonging to one of these groups with an amino acid belonging to any of the other groups. More conservative substitution may be performed by replacing an amino acid belonging to one of these groups with another amino acid belonging to the same group. Likewise, the deletion or the substitution in an amino acid sequence may be appropriately performed.

### 5. Preparation

The therapeutic agent for rheumatoid arthritis of the present invention comprises a therapeutically effective amount of a drug that inhibits FKN-CX3CR1 interaction. The amount of the drug that inhibits FKN-CX3CR1 interaction can vary according to the type of the drug, a recipient, an administration route, a dosing interval, etc.

Hereinafter, the therapeutic agent for rheumatoid arthritis of the present invention, comprising an anti-FKN antibody as an active ingredient will be described as an example.

The therapeutically effective amount of the anti-FKN antibody (e.g., H3-2L4) is, but not limited to, 50 mg to 1000 mg, 100 mg to 800 mg, 200 mg to 800 mg, or 400 to 800 mg per dose in subcutaneous administration. In a particular embodiment of the present invention, the anti-FKN antibody (e.g., H3-2L4) is administered in an amount of at least 50 mg, at least 100 mg, at least 150 mg, at least 200 mg, at least 250 mg, at least 300 mg, at least 350 mg, at least 400 mg, at least 450 mg, at least 500 mg, at least 550 mg, at least 600 mg, at least 650 mg, at least 700 mg, at least 750 mg, or at least 800 mg. In a further alternative embodiment, the anti-FKN antibody (e.g., H3-2L4) is administered in an amount of 100 mg, 200 mg, 400 mg, 600 mg or 800 mg.

The dosage form of the therapeutic agent for rheumatoid arthritis of the present invention is not particularly limited and is typically a preparation for injection prepared for subcutaneous administration. The therapeutic agent for rheumatoid arthritis of the present invention can be prepared, for example, as a preparation for injection of the anti-FKN antibody, together with a pharmaceutically acceptable excipient, in injectable water, physiological saline, or phosphate-buffered saline without limitations. Examples of the pharmaceutically acceptable excipient used in the present invention include, but are not limited to, a stabilizer, a surfactant, and a preservative.

The stabilizer used in the present invention is, for example, a carbohydrate, a saccharide, or a sugar (e.g., sucrose) accepted by an authority as an appropriate additive or excipient for pharmaceutical preparations. The concentration of the stabilizer is 15 to 250 mM, 150 to 250 mM, or 200 mM. The preparation may contain a secondary stabilizer.

Appropriate examples of the pharmaceutically acceptable surfactant used in the present invention include, but are not limited to, polyoxyethylene sorbitan fatty acid ester (Tween), polyethylene polypropylene glycol, polyoxyethylene stearate, polyoxyethylene alkyl ether (e.g., polyoxyethylene monolauryl ether), alkylphenyl polyoxyethylene ether (Triton-X), polyoxyethylene-polyoxypropylene copolymers (Poloxamer and Pluronic), and sodium dodecyl sulfate (SDS). The most appropriate polyoxyethylene sorbitan fatty acid esters are polysorbate 20 (Tween 20) and polysorbate 80 (Tween 80). The concentration of the surfactant is 0.01 to 0.1% (w/v), 0.01 to 0.08% (w/v), or 0.025 to 0.075% (w/v), for example, 0.05% (w/v).

Examples of the preservative used in the present invention include, but are not limited to, paraben, benzyl alcohol, sodium benzoate, phenol, benzalkonium chloride, thimerosal, chlorobutanol, benzoic acid, sodium bisulfite, sodium propionate, and arbitrary combinations or mixtures thereof.

Examples of the buffer used in the present invention include, but are not limited to, histidine, citrate, phosphate, glycine, acetate, and arbitrary combinations or mixtures thereof.

The preparation of the present invention may contain a buffer or a pH adjuster for controlling pH. In one embodiment, the preparation of the present invention has a pH in the range of 4.0 to 9.0, in the range of 5.0 to 9.0, in the range of 5.0 to 8.0, in the range of 5.0 to 7.5, in the range of 5.5 to 7.0, or in the range of 5.5 to 6.5.

The therapeutic agent for rheumatoid arthritis of the present invention can be isotonic to human blood, i.e., the therapeutic agent for rheumatoid arthritis of the present invention can have essentially the same osmotic pressure as that of human blood. Such an isotonic preparation generally has an osmotic pressure of 250 mOSm to 350 mOSm. The isotonicity can be measured by use of, for example, a vapor pressure or ice freezing-type osmometer. Examples of the tonicity agent used in the present invention include, but are not limited to, saccharides, salts, and amino acids.

In addition to the excipients mentioned above, typical excipients and processes for the production of the preparation for injection for subcutaneous administration are known in the art. See, for example, Introduction to Pharmaceutical Dosage Forms, 1985, Ansel, H.C., Lea and Febiger, Philadelphia, Pa.; Remington's Pharmaceutical Sciences, 1995, Mack Publ. Co., Easton, Pa. This literature is incorporated herein by reference in its entirety.

In one embodiment, the therapeutic agent for rheumatoid arthritis comprising the anti-FKN antibody (e.g., H3-2L4) can adopt a pharmaceutical formulation described in Japanese Patent Laid-Open No. 2017-193541.

In one embodiment of the present invention, the anti-FKN antibody (e.g., H3-2L4) is used such that at least 100 mg of the anti-FKN antibody per dose is subcutaneously administered to a human. In another embodiment of the present invention, the therapeutic agent for rheumatoid arthritis of the present invention is used such that 100 mg to 400 mg of the anti-FKN antibody per dose is subcutaneously administered to a human. In one embodiment of the present invention, the therapeutic agent for rheumatoid arthritis of the present invention is used such that 100 mg, 200 mg or 400 mg of the anti-FKN antibody per dose is subcutaneously administered to a human. In another embodiment of the present invention, the therapeutic agent for rheumatoid arthritis of the present invention is used such that 200 mg to 400 mg of the anti-FKN antibody per dose is subcutaneously administered to a human.

In one embodiment of the present invention, the anti-FKN antibody (e.g., H3-2L4) is used such that at least 200 mg of the anti-FKN antibody per dose is subcutaneously administered to a human. In another embodiment of the present invention, the therapeutic agent for rheumatoid arthritis of the present invention is used such that 200 mg to 600 mg of the anti-FKN antibody per dose is subcutaneously administered to a human. In one embodiment of the present invention, the therapeutic agent for rheumatoid arthritis of the present invention is used such that 200 mg, 400 mg, or 600 mg of the anti-FKN antibody per dose is subcutaneously administered to a human. In another embodiment of the present invention, the therapeutic agent for rheumatoid arthritis of the present invention is used such that 400 mg to 600 mg of the anti-FKN antibody per dose is subcutaneously administered to a human.

In one embodiment of the present invention, the anti-FKN antibody (e.g., H3-2L4) is used such that at least 400 mg of the anti-FKN antibody per dose is subcutaneously administered to a human. In another embodiment of the present invention, the therapeutic agent for rheumatoid arthritis of the present invention is used such that 400 mg to 800 mg of the anti-FKN antibody per dose is subcutaneously administered to a human. In one embodiment of the present invention, the therapeutic agent for rheumatoid arthritis of the present invention is used such that 400 mg, 600 mg, or 800 mg of the anti-FKN antibody per dose is subcutaneously administered to a human. In another embodiment of the present invention, the therapeutic agent for rheumatoid arthritis of the present invention is used such that 400 mg to 600 mg of the anti-FKN antibody per dose is subcutaneously administered to a human. In an alternative embodiment of the present invention, the therapeutic agent for rheumatoid arthritis of the present invention is used such that 600 mg to 800 mg of the anti-FKN antibody per dose is subcutaneously administered to a human.

The number of doses and dosing interval of the therapeutic agent for rheumatoid arthritis of the present invention can vary according to the amount of the anti-FKN antibody administered per dose, and an administration route, etc.

The dosing interval of the anti-FKN antibody (e.g., H3-2L4) is, for example, once a week to once every two months, once a week to once a month, once a week to once every two weeks, once a week, or once every two weeks, and these dosing intervals can be combined.

In one embodiment of the present invention, the anti-FKN antibody (e.g., H3-2L4) is used such that 100 mg to 400 mg of the anti-FKN antibody per dose is subcutaneously administered a plurality of times to a human in need thereof at dosing intervals of once a week to once every two months. In another embodiment of the present invention, the therapeutic agent for rheumatoid arthritis of the present invention is used such that 100 mg to 400 mg of the anti-FKN antibody per dose is subcutaneously administered a plurality of times to a human in need thereof at dosing intervals of once a week to once a month. In an alternative embodiment of the present invention, the therapeutic agent for rheumatoid arthritis of the present invention is used such that 100 mg to 400 mg of the anti-FKN antibody per dose is subcutaneously administered a plurality of times to a human in need thereof at dosing intervals of once a week to once every two weeks. In a particular embodiment of the present invention, the therapeutic agent for rheumatoid arthritis of the present invention is used such that 100 mg to 400 mg of the anti-FKN antibody per dose is subcutaneously administered at dosing intervals of every other week after two once-a-week administrations.

In one embodiment of the present invention, the anti-FKN antibody (e.g., H3-2L4) is used such that 400 mg to 800 mg of the anti-FKN antibody per dose is subcutaneously administered a plurality of times to a human in need thereof at dosing intervals of once a week to once every two months. In another embodiment of the present invention, the therapeutic agent for rheumatoid arthritis of the present invention is used such that 400 mg to 800 mg of the anti-FKN antibody per dose is subcutaneously administered a plurality of times to a human in need thereof at dosing intervals of once a week to once a month. In an alternative embodiment of the present invention, the therapeutic agent for rheumatoid arthritis of the present invention is used such that 400 mg to 800 mg of the anti-FKN antibody per dose is subcutaneously administered a plurality of times to a human in need thereof at dosing intervals of once a week to once every two weeks. In a particular embodiment of the present invention, the therapeutic agent for rheumatoid arthritis of the present invention is used such that 400 mg to 800 mg of the anti-FKN antibody per dose is subcutaneously administered at dosing intervals of every other week after two once-a-week administrations.

In one embodiment of the present invention, 100 mg, 200 mg, or 400 mg of the anti-FKN antibody (e.g., H3-2L4) per dose is subcutaneously administered a plurality of times to a human in need thereof at dosing intervals of once a week to once every two months. In another embodiment of the present invention, 100 mg, 200 mg, or 400 mg of the anti-FKN antibody per dose is subcutaneously administered a plurality of times to a human in need thereof at dosing intervals of once a week to once a month. In an alternative embodiment of the present invention, 100 mg, 200 mg, or 400 mg of the anti-FKN antibody per dose is subcutaneously administered a plurality of times to a human in need thereof at dosing intervals of once a week to once every two weeks. In a particular embodiment of the present invention, 100 mg, 200 mg, or 400 mg of the anti-FKN antibody per dose is subcutaneously administered at dosing intervals of every other week after two once-a-week administrations.

In one embodiment of the present invention, 400 mg, 600 mg, or 800 mg of the anti-FKN antibody (e.g., H3-2L4) per dose is subcutaneously administered a plurality of times to a human in need thereof at dosing intervals of once a week to once every two months. In another embodiment of the present invention, 400 mg, 600 mg, or 800 mg of the anti-FKN antibody per dose is subcutaneously administered a plurality of times to a human in need thereof at dosing intervals of once a week to once a month. In an alternative embodiment of the present invention, 400 mg, 600 mg, or 800 mg of the anti-FKN antibody (e.g., H3-2L4) per dose is subcutaneously administered a plurality of times to a human in need thereof at dosing intervals of once a week to once every two weeks. In a particular embodiment of the present invention, 400 mg, 600 mg, or 800 mg of the anti-FKN antibody (e.g., H3-2L4) per dose is subcutaneously administered at dosing intervals of every other week after two once-a-week administrations.

The amount of the FKN-CX3CR1 interaction inhibitor administered per dose can be increased or decreased after a lapse of a given period after the start of administration. The timing of increase or decrease in the amount is, for example, 1 week, 2 weeks, 3 weeks, 4 weeks, 5 weeks, 6 weeks, 7 weeks, 8 weeks, 9 weeks, 10 weeks, 12 weeks, 16 weeks, 20 weeks, 24 weeks, 28 weeks, 32 weeks, 36 weeks, 40 weeks, 44 weeks, 48 weeks, 52 weeks, 58 weeks, 60 weeks, 64 weeks, 68 weeks, 72 weeks, 76 weeks, 80 weeks, 80 weeks, 88 weeks, 92 weeks, 100 weeks, 104 weeks, 108 weeks, 112 weeks or 116 weeks after the start of administration. In one embodiment, the timing of increase or decrease in the amount is 12 weeks or 24 weeks after the start of treatment. In one embodiment, the dose after increase or decrease in the amount is 1/3 to 3/4, 1/3 to 2/3, or 1/2 to 2/3 of that at the start of administration. In one embodiment, the increase or decrease in the amount is performed zero times, once, or a plurality of times.

In one embodiment of the present invention, the anti-FKN antibody (e.g., H3-2L4) is administered in an amount of 400 mg per dose at weeks 0, 1 and 2 and then subcutaneously administered at dosing intervals of every other week, and the amount is decreased to 200 mg at dosing intervals of every other week at the time of 12 weeks after the start of administration.

In one embodiment of the present invention, the anti-FKN antibody (e.g., H3-2L4) is administered in an amount of 400 mg per dose at weeks 0, 1 and 2 and then subcutaneously administered at dosing intervals of every other week, and the amount is decreased to 200 mg at dosing intervals of every other week at the time of 24 weeks after the start of administration.

In one embodiment of the present invention, the anti-FKN antibody (e.g., H3-2L4) is subcutaneously administered in an amount of 400 mg per dose at dosing intervals of every other week up to week 10, and the amount is decreased to 200 mg at dosing intervals of every other week at the time of 12 weeks after the start of administration.

In one embodiment of the present invention, the anti-FKN antibody (e.g., H3-2L4) is subcutaneously administered in an amount of 400 mg per dose at dosing intervals of every other week up to week 10, and the amount is decreased to 200 mg at dosing intervals of every other week at the time of 24 weeks after the start of administration.

In one embodiment of the present invention, the anti-FKN antibody (e.g., H3-2L4) is subcutaneously administered in an amount of 600 mg per dose at dosing intervals of every other week up to week 10, and the amount is decreased to 400 mg at dosing intervals of every other week at the time of 12 weeks after the start of administration.

In one embodiment of the present invention, the anti-FKN antibody (e.g., H3-2L4) is subcutaneously administered in an amount of 600 mg per dose at dosing intervals of every other week up to week 10, and the amount is decreased to 400 mg at dosing intervals of every other week at the time of 24 weeks after the start of administration.

In one embodiment of the present invention, the anti-FKN antibody (e.g., H3-2L4) is subcutaneously administered in an amount of 600 mg per dose at dosing intervals of every other week up to week 10, and the amount is decreased to 200 mg at dosing intervals of every other week at the time of 12 weeks after the start of administration.

In one embodiment of the present invention, the anti-FKN antibody (e.g., H3-2L4) is subcutaneously administered in an amount of 600 mg per dose at dosing intervals of every other week up to week 10, and the amount is decreased to 200 mg at dosing intervals of every other week at the time of 24 weeks after the start of administration.

In one embodiment of the present invention, the anti-FKN antibody (e.g., H3-2L4) is subcutaneously administered in an amount of 800 mg per dose at dosing intervals of every other week up to week 10, and the amount is decreased to 600 mg at dosing intervals of every other week at the time of 12 weeks after the start of administration.

In one embodiment of the present invention, the anti-FKN antibody (e.g., H3-2L4) is subcutaneously administered in an amount of 800 mg per dose at dosing intervals of every other week up to week 10, and the amount is decreased to 600 mg at dosing intervals of every other week at the time of 24 weeks after the start of administration.

In one embodiment of the present invention, the anti-FKN antibody (e.g., H3-2L4) is subcutaneously administered in an amount of 800 mg per dose at dosing intervals of every other week up to week 10, and the amount is decreased to 400 mg at dosing intervals of every other week at the time of 12 weeks after the start of administration.

In one embodiment of the present invention, the anti-FKN antibody (e.g., H3-2L4) is subcutaneously administered in an amount of 800 mg per dose at dosing intervals of every other week up to week 10, and the amount is decreased to 400 mg at dosing intervals of every other week at the time of 24 weeks after the start of administration.

Those skilled in the art should understand that the present invention may be carried out by any one of or appropriate combination of two or more of all aspects described in the present specification unless a technical contradiction arises. Further, those skilled in the art should understood that the present invention can be preferably carried out by an appropriate combination of all preferred or advantageous aspects described in the present specification unless a technical contradiction arises.

Literatures cited in the present specification should be interpreted as being incorporated herein by reference in their entirety. Those skilled in the art can understand related contents disclosed in these literatures by reference as a part of the present specification without departing from the spirit and scope of the present invention according to the context of the present specification.

Literatures cited in the present specification are provided merely for the purpose of disclosing related techniques before the filing date of the present application. It should not be understood that the present inventors admit to having no right preceding such disclosure due to the prior inventions or any other reasons. All statements of these literatures are based on information which has been available by the present applicant, and there is no admission that the contents of these statements are accurate.

The terms in the present specification are used for illustrating particular embodiments and are not intended to limit the invention.

The term "comprise" or "include" used in the present specification means that described items (members, steps, factors, numbers, etc.) are present and the presence of the other items (members, steps, factors, numbers, etc.) is not excluded therefrom, unless the context evidently requires different interpretation. The term "consist of" encompasses aspects described by the terms "consist of" and/or "consist essentially of".

All terms (including technical terms and scientific terms) used herein have the same meanings as those understood in a broad sense by those skilled in the art to which the present invention belongs, unless otherwise defined. The terms used herein should be interpreted as having meanings consistent with meanings in the present specification and related technical fields and should not be interpreted in an idealized or excessively formal sense, unless otherwise defined.

Terms such as "first" or "second" are used for expressing various factors. However, these factors are understood to be not limited by these terms themselves. These terms are used merely for differentiating one factor from the other factors. For example, the first factor may be described as the second factor, and vice versa, without departing from the scope of the present invention.

In the present specification and the scope of claims, it should be understood that numerical values used for indicating component contents, numerical ranges, etc., are modified with the term "approximately" unless otherwise specified. For example, "10 µg" is interpreted as meaning "approximately 10 µg" unless otherwise specified. Those skilled in the art can naturally understand the extent thereof rationally according to the technical common sense and the context of the present specification.

It should be understood that each aspect indicated in a singular form used in the present specification and claims may be in a plural form, and vice versa, unless the context evidently requires different interpretation and unless a technical contradiction arises.

Hereinafter, the present invention will be described in more detail with reference to Examples. However, the present invention can be embodied by various aspects and is not intended to be limited by Examples described herein. Those skilled in the art can implement the present invention by various changes or modifications, additions, deletions, substitutions, etc., without departing from the spirit or scope of the present invention.

### [Examples]

### Example 1: Preparation of humanized anti-human fractalkine antibody

Humanized anti-human fractalkine antibody H3-2L4 was used in administration tests to humans given below. The preparation of H3-2L4 including humanization was performed as described in WO2011/052799. H3-2L4 used in Example 2 or later was prepared by the method described in Japanese Patent Laid-Open No. 2017-193541.

The sequence of H3-2L4 is as given below. The constant regions of H3-2L4 are the constant regions of human IgG2 containing two mutations (V234A and G237A) inserted in the amino acid sequence.
(1) Full-length heavy chain of H3-2L4 (SEQ ID NO: 1) 
QVQLVQSGAEVKKPGASVKVSCKASGYTFTNYYIHWVKQAPGQGLEWIGWIYPGDGS
PKFNERFKGRTTLTADKSTNTAYMLLSSLRSEDTAVYFCATGPTDGDYFDYWGQGTT
VTVSSASTKGPSVFPLAPCSRSTSESTAALGCLVKDYFPEPVTVSWNSGALTSGVHT
FPAVLQSSGLYSLSSVVTVPSSNFGTQTYTCNVDHKPSNTKVDKTVERKCCVECPPC
PAPPAAAPSVFLFPPKPKDTLMISRTPEVTCVVVDVSHEDPEVQFNWYVDGVEVHNA
KTKPREEQFNSTFRVVSVLTVVHQDWLNGKEYKCKVSNKGLPAPIEKTISKTKGQPR
EPQVYTLPPSREEMTKNQVSLTCLVKGFYPSDIAVEWESNGQPENNYKTTPPMLDSD
GSFFLYSKLTVDKSRWQQGNVFSCSVMHEALHNHYTQKSLSLSPGK
(2) Full-length light chain of H3-2L4 (SEQ ID NO: 2) 
DIQMTQSPSSLSASVGDRVTITCRASGNIHNFLAWYQQKPGKAPKLLIYNEKTLADG
VPSRFSGSGSGTDYTLTISSLQPEDFATYFCQQFWSTPYTFGGGTKVEIKRTVAAPS
VFIFPPSDEQLKSGTASVVCLLNNFYPREAKVQWKVDNALQSGNSQESVTEQDSKDS
TYSLSSTLTLSKADYEKHKVYACEVTHQGLSSPVTKSFNRGEC
(3) Heavy chain variable region of H3-2L4 (SEQ ID NO: 3) 
QVQLVQSGAEVKKPGASVKVSCKASGYTFTNYYIHWVKQAPGQGLEWIGWIYPGDGS

PKFNERFKGRTTLTADKSTNTAYMLLSSLRSEDTAVYFCATGPTDGDYFDYWGQGTT

VTVSS
(4) Light chain variable region of H3-2L4 (SEQ ID NO: 4) 
DIQMTQSPSSLSASVGDRVTITCRASGNIHNFLAWYQQKPGKAPKLLIYNEKTLADG

VPSRFSGSGSGTDYTLTISSLQPEDFATYFCQQFWSTPYTFGGGTKVEIK
(5) Amino acid sequences of CDR-H1 to CDR-H3 and CDR-L1 to CDR-L3 of H3-2L4:
   CDR-H1 of H3-2L4 (SEQ ID NO: 5)
   NYYIH
   CDR-H2 of H3-2L4 (SEQ ID NO: 6)
   WIYPGDGSPKFNERFKG
   CDR-H3 of H3-2L4 (SEQ ID NO: 7)
   GPTDGDYFDY
   CDR-L1 of H3-2L4 (SEQ ID NO: 8)
   RASGNIHNFLA
   CDR-L2 of H3-2L4 (SEQ ID NO: 9)
   NEKTLAD
   CDR-L3 of H3-2L4 (SEQ ID NO: 10)
   QQFWSTPYT

### Example 2: Phase 1/2 clinical trial by repeated subcutaneous administration

A phase 1/2 clinical trial targeting rheumatoid arthritis patients was conducted for evaluating safety and tolerability by repeated subcutaneous administrations of H3-2L4.

### 2-1. Clinical trial design

This clinical trial is a multicenter, open-label, uncontrolled, multiple-ascending dose (MAD) study targeting Japanese patients with rheumatoid arthritis and having a primary object to evaluate safety and tolerability by repeated subcutaneous administrations of H3-2L4 for 12 weeks. This clinical trial involved a total of 37 cases including 12 cases in a 100 mg group, 15 cases in a 200 mg group, and 10 cases in a 400 mg group. Among them, 28 cases entered to a extension phase up to week 52. For ascending doses, tests were sequentially conducted from lower doses and entered to higher-dose administration groups after safety evaluation.

This clinical trial was composed of a screening phase, an observation phase, a treatment phase, a extension phase, and a follow-up phase.

The screening test was carried out 42 days to 2 days before the start of administration of a study drug, and the test for the observation phase was carried out on the day before initial administration of the study drug or before the administration on this day. H3-2L4 was administered to the subjects confirmed to be eligible. Criteria for the eligibility were as follows:
(1) those at age 20 years or older and younger than 65 years;
(2) rheumatoid arthritis patients who meet the 1987 ACR Classification Criteria or the 2010 ACR/EULAR Classification Criteria;
(3) patients who received either or both of the following treatments prior to the start of screening and present 4 or more tender joints (in 68 joints) and 4 or more swollen joints (in 66 joints) by evaluation in the screening phase and the observation phase:
   - patients who received 3-month or longer treatment with methotrexate (MTX). In patients who had to discontinue the administration due to adverse reaction, such history of treatment for 3 months or longer is not required, and
   - patients who received 3-month or longer treatment with an anti-TNF drug. However, the anti-TNF drug used in the treatment is limited to any one of adalimumab, infliximab, golimumab, certolizumab pegol, and etanercept (the anti-TNF drug includes its biosimilar);
(4) patients who have not received any biologics (tocilizumab, abatacept, etc.) other than the anti-TNF drug, or two or more anti-TNF drugs in the past;
(5) patients who have 0.6 mg/dL or higher of high sensitive CRP (hs-CRP) or an erythrocyte sedimentation rate (ESR) of 28 mm/hr or higher at the screening phase;
(6) patients who, in the case of using disease-modifying antirheumatic drugs (DMARDs) other than MTX, bucillamine or salazosulfapyridine, are capable of discontinuing administration 4 weeks or more before the start of administration of the study drug. However, use of any one of MTX, bucillamine and salazosulfapyridine is allowed from 4 weeks before the start of administration of the study drug through the clinical trial period;
(7) patients who, in the case of using adrenocortical steroid in an amount of 10 mg/day in terms of prednisolone, are capable of decreasing the amount to 10 mg/day or less in terms of prednisolone 4 weeks or more before the start of administration of the study drug; and
(8) patients who have a body weight of 30 kg or higher and 100 kg or lower at the time of screening.

### 2-2: Administration of study drug

The types and formulations of the study drugs used in this clinical trial are as follows.

**[Table 1]**

| | | |
|---|---|---|
| Table 1 Type of study drug | | |

| Type | Dosage form and content | Manufacturer |
|---|---|---|
| H3-2L4 | Aqueous solution containing 100 mg of H3-2L4 in one vial (1 mL) | Eisai Co., Ltd. |

**[Table 2]**

| | |
|---|---|
| Table 2 Formulation | |

| Component | Concentration |
|---|---|
| H3-2L4 | 100 mg/mL |
| Phosphate buffer solution | 25 mM |
| Sucrose | 200 mM |
| Glycine | 50 mM |
| Polysorbate 80 | 0.05% |

In the treatment phase, H3-2L4 was administered a total of 7 times (week 0, week 1, week 2, and subsequently, every two weeks until week 10).

For the 100 mg administration group, subcutaneous administration was performed to any one of the right and left upper arms, the right and left flanks, and the right and left femoral regions. For the 200 mg administration group, 1 mL was subcutaneously administered to each of any two of these sites. For the 400 mg administration group, 1 mL was subcutaneously administered to each of any four of these sites. As for the 400 mg administration group, 2 mL was able to be subcutaneously administered to each of any two of the sites as long as a principal investigator or a subinvestigator judged that this subcutaneous administration was properly achieved. The administration of the study drug was carried out after the completion of all investigations (except for findings about administration sites) scheduled on that day.

The same dose was further administered 20 times every two weeks (40 weeks) to subjects who had no problem with safety in evaluation 2 weeks after the completion of the 7th administration (at week 12), exhibited 20% or more improvement in both of the tender joint count and the swollen joint count from the observation phase, and desired continuous administration (extension phase). As for the extension phase of the 400 mg administration group, the dose was able to be decreased to 200 mg (which was also able to be brought back to 400 mg thereafter) in the judgment of a principal investigator or a subinvestigator. The subjects who enter the extension phase were observed and investigated until 52 weeks after the initial administration. The dosing intervals of the study drug were set to 7 days or longer (6-day or longer resting period). If a dosing interval was shorter than 7 days because of changing the day of observation and investigation, only the observation and investigation were carried out with a cessation of administration of the study drug.

### 2-3: Effectiveness

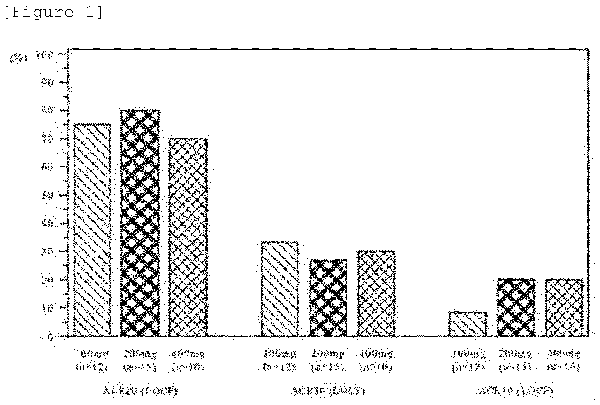

In this clinical trial, effectiveness was exploratorily evaluated. The ACR20 response rate after week 12 was 75.0% (9/12 cases) for the 100 mg group, 80.0% (12/15 cases) for the 200 mg group, and 70.0% (7/10 cases) for the 400 mg group. The ACR50 response rate was 33.3% (4/12 cases) for the 100 mg group, 26.7% (4/15 cases) for the 200 mg group, and 30.0% (3/10 cases) for the 400 mg group. The ACR70 response rate was 8.3% (1/12 cases) for the 100 mg group, 20.0% (3/15 cases) for the 200 mg group, and 20.0% (2/10 cases) for the 400 mg group (Figure 1). Data was complemented by last observation carried forward (LOCF) as to cases found to have missing effectiveness data at each time of evaluation because of early discontinuation or other reasons.

### 2-4: Subgroup analysis based on CD16+ monocyte ratio

The ratio of CD16+ monocytes to total monocytes in peripheral blood collected before administration (day 1) (baseline) counted starting at the administration start day (day 1 (week 0) was measured by use of flow cytometry.

Blood collected with Cyto-Chex BCT (Streck, Inc., 213386) and BD Pharm Lyse (BD Biosciences, 555899)) diluted 10-fold with a lysis buffer (Otsuka Distilled Water (Otsuka Pharmaceutical Factory, Inc.) were mixed at 1:9 (volume ratio), then mixed by inversion several times, and left at room temperature for 10 minutes to hemolyze red blood cells. The hemolyzed sample was centrifuged under conditions of room temperature, 290 x g, and 5 minutes. Residual cells were washed with a FACS buffer (buffer prepared as PBS containing 1 mM EDTA and 1% FBS using 0.5 M EDTA (Invitrogen Corp., 15575-038), FBS (fetal bovine serum, Hyclone Laboratories Inc., SH30396.03) and PBS (Santa Cruz Biotechnology, Inc., sc-296028). The residual cells were suspended in the same amount of a FACS buffer as that of the hemolyzed blood to prepare a cell suspension. For blocking, the cell suspension and FcR Blocking Reagent (Miltenyi Biotec, 130-059-901) were mixed at 10:2 (volume ratio) and left on ice for 30 minutes. The cell suspension was added to a 96-well plate. The cells were centrifuged under conditions of 4°C, 290 x g, and 5 minutes. After discarding of the supernatant, a mixed antibody solution (fluorescently labeled anti-CD14 antibody (Alexa Fluor 647-CD14, BioLegend, Inc., 325612) and fluorescently labeled anti-CD16 antibody (FITC-CD16, Abcam plc, ab115920) adjusted with a FACS buffer) was added to the cells, and the plate was stirred in a plate shaker and left on ice for 30 minutes in the dark to stain the cells. A FACS buffer was added to the cells thus stained, which were then washed by centrifugation under conditions of 4°C, 290 x g, and 5 minutes. The cells were suspended in 200 µL of a FACS buffer. Sample data was obtained using BD FACSCanto II (Becton, Dickinson and Company, 338962).

The sample data obtained in BD FACSCanto II was exported to a fcs file. The fsc file was analyzed with FlowJo (FLOWJO, LLC). In FSC-A and SSC-A development, peripheral blood-derived mononuclear cells (PBMC) were fractionated into monocytes. The monocytes were developed with CD14 and CD16 and fractionated into CD16+ monocytes. The ratio (percentage) of the CD16+ monocytes to total monocytes was calculated.

The relationship between the obtained baseline CD16+ monocyte ratio and the ACR20, ACR50 and ACR70 responses was evaluated. In the evaluation of ACR, as in the section 2-3, data was complemented by last observation carried forward (LOCF) as to cases found to have missing effectiveness data at each time of evaluation because of early discontinuation or other reasons.

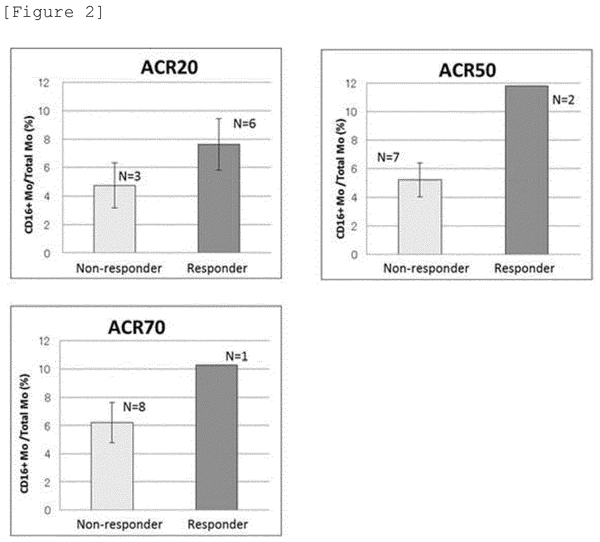

In the 400 mg group at 12 weeks after the start of administration, the average values of baseline CD16+ monocyte ratios of ACR20, ACR50 and ACR70 responders and non-responders were 7.6% (N = 6) for the ACR20 responders, 4.7% (N = 3) for the ACR20 non-responders, 11.8% (N = 2) for the ACR50 responders, 5.2% (N = 7) for the ACR50 non-responders, 10.3% (N = 1) for the ACR50 responders, and 6.2% (N = 8) for the ACR50 non-responders (Figure 2). This result suggested the relationship between high CD16+ monocyte ratios and effectiveness.

### Example 3: Dose response study of H3-2L4 in participants with rheumatoid arthritis inadequately responding to methotrexate (phase 2 clinical trial)

### 3-1. Clinical trial design

This clinical trial is a multicenter, randomized, double-blind, placebo-controlled, parallel-group study. In this clinical trial, four groups were set: H3-2L4 100 mg, 200 mg, 400 mg, and placebo groups. For the H3-2L4 100 mg, 200 mg and placebo groups, administration was performed at weeks 0, 1 and 2 and thereafter, every 2 weeks. For the H3-2L4 400 mg group, H3-2L4 was administered in an amount of 400 mg at weeks 0, 1, 2, 4, 6, 8 and 10 and thereafter, in an amount of 200 mg every 2 weeks.

This clinical trial was composed of s screening phase, an observation phase, an treatment phase, a extension phase and a follow-up phase.

The screening test was carried out within 42 days before the start of administration of a study drug. The subjects confirmed to be eligible in the observation phase were targeted and allocated to any of the H3-2L4 100 mg, 200 mg, 400 mg and placebo groups at 1:2:2:2 using dynamic allocation with CRP values in the screening phase, disease durations, and histories of administration of biological products as allocation factors. Criteria for the eligibility were as follows:
(1) patients at age 18 years or older and younger than 75 years;
(2) RA patients who meet the 1987 ACR Classification Criteria or the 2010 ACR/EULAR Classification Criteria 12 weeks or more before obtainment of consent;
(3) patients who received treatment with MTX for 12 weeks or longer within the range of 6 mg/ weeks to 16 mg/ weeks before the start of screening and presented 6 or more tender joints (in 68 joints) and 6 or more swollen joints (in 66 joints) by evaluation in the screening phase and the observation phase;
(4) patients who are able to continuously use MTX in given dosage and administration (6 mg/ weeks to 16 mg/ weeks) from 4 weeks or more before the start of administration of the study drug to the completion of the extension phase (or discontinuation);
(5) For patients with a history of biologics treatment for RA (including a treatment history in clinical studies), the following criteria should be fulfilled:
   - the history of biologics treatment for RA with any of adalimumab, infliximab, golimumab, certolizumab pegol, etanercept, tocilizumab, and abatacept (including biosimilars);
   - No biologics treatment for RA within 12 weeks prior to the study treatment;
(6) patients who have 0.6 mg/dL or higher of CRP or an erythrocyte sedimentation rate (ESR) of 28 mm/hr or higher in the screening phase;
(7) patients who have 3 or more bone erosions in joint X-ray images in the screening phase, or patients who are positive to a rheumatoid factor (RF) or an anti-cyclic citrullinated peptide (CCP) antibody and have one or more bone erosions; and
(8) patients who have a body weight of 30 kg or higher and 100 kg or lower in the screening phase.

### 3-2: Administration of study drug

The types and formulations of the study drugs used in this clinical trial are as follows.

**[Table 3]**

| | |
|---|---|
| Table 3 Type of study drug | |

| Study drug | Content and dosage form |
|---|---|
| H3-2L4 | Aqueous solution containing 50 mg or 100 mg of H3-2L4 in one vial (1 mL) |
| Placebo | Aqueous solution containing no H3-2L4 in one vial (1 mL) |
| Preservation conditions: preserved at 2 to 8°C in the dark | |
| Manufacturer: Eisai Co., Ltd. | |

**[Table 4]**

| | | | |
|---|---|---|---|
| Table 4 Formulation | | | |

| Component | Concentration | | |
|---|---|---|---|
| | H3-2L4 100 mg/ml | H3-2L4 50 mg/mL | Placebo |
| H3-2L4 | 100 mg/mL | 50 mg/mL | - |
| Phosphate buffer solution (pH 5.8) | 25 mM | 25 mM | 25 mM |
| Sucrose | 200 mM | 200 mM | 200 mM |
| Glycine | 50 mM | 50 mM | 50 mM |
| Polysorbate 80 | 0.05% | 0.05% | 0.05% |

The treatment phase was set to 24 weeks. H3-2L4 or placebo was administered at weeks 0, 1 and 2 and thereafter, every 2 weeks up to week 22 in a double-blinded manner.

Subjects who completed evaluation at week 24 in the treatment phase entered to the extension phase. The extension phase was set to 104 weeks after the start of administration of the study drug. H3-2L4 was administered in an amount of 200 mg up to week 102 every 2 weeks in an open-label manner. Only one increase in amount (400 mg was administered 6 times every 2 weeks) was also accepted when the effect was inadequate or at the time of exacerbation (when no improvement in any of the numbers of tender joints and swollen joints was found in two consecutive evaluations at an interval of 1 week or longer, as compared with the observation phase) in the extension phase. The administration of the study drug was carried out after the completion of all investigations (except for findings about administration sites) scheduled on that day.

In this clinical trial, a study drug containing 50 mg or 100 mg of H3-2L4 or placebo in one vial (1 mL) was used. In treatment phase, the study drug was subcutaneously administered in an amount of 4 mL at weeks 0 to 10 and in an amount of 2 mL at weeks 12 to 22. In the extension phase, the study drug was subcutaneously administered in an amount of 2 mL for the administration of 200 mg of H3-2L4 and in an amount of 4 mL for the administration of 400 mg of H3-2L4. At the time of the 2 mL administration, 1 mL was subcutaneously administered to each of any two of the right and left upper arms, the right and left flanks, and the right and left femoral regions. At the time of the 4 mL administration, 1 mL was subcutaneously administered to each of any four of the right and left upper arms, the right and left flanks, and the right and left femoral regions. As for the 4 mL administration, 2 mL was able to be subcutaneously administered to each of any two of the sites as long as a principal investigator or a subinvestigator judged that this subcutaneous administration was properly achieved.

The evaluation started at week 0 and carried out within 3 days pre- or post-administration at weeks 1 to 12 and within 7 days pre-or post-administration at week 14 or later. In the follow-up phase, the evaluation was carried out within 7 days before or after the prescribed day ("70 days after final administration" means "within 7 days post-administration"). However, the dosing intervals of the study drug were set to 3 days or more up to 2 weeks from the start of administration and 6 days or more at 4 weeks or later from the start of administration. If the scheduled evaluation and administration of the study drug were unable to be carried out within this range, the administration of the study drug was regarded as drug holiday though each evaluation was carried out.

This clinical trial involved a total of 190 cases including 54 cases in the placebo group, 28 cases in the H3-2L4 100 mg group, 54 cases in the 200 mg group, and 54 cases in the 400/200 mg group. Among them, 169 cases completed the treatment phase.

### 3-3: Effectiveness

ACR20, ACR50 and ACR70 response rates, etc. were evaluated at each time of evaluation on a dose basis.

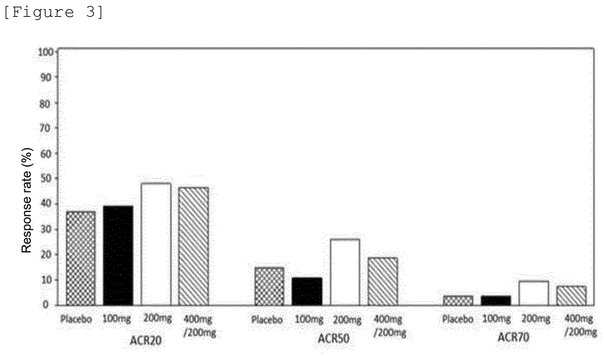

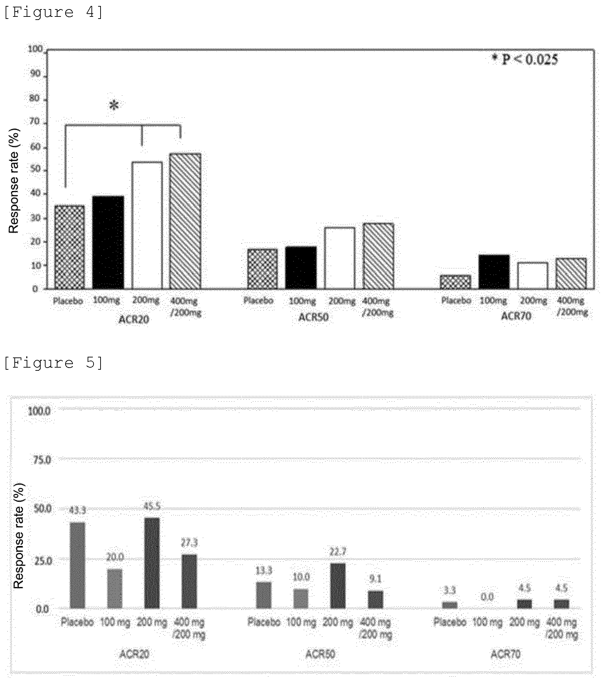

The ACR20 response rate after week 12 was 37.0% (20/54 cases) for the placebo group, 39.3% (11/28 cases) for the 100 mg group, 48.1% (26/54 cases) for the 200 mg group, and 46.3% (25/54 cases) for the 400/200 mg group. The ACR50 response rate was 14.8% (8/54 cases) for the placebo group, 10.7% (3/28 cases) for the 100 mg group, 25.9% (14/54 cases) for the 200 mg group, and 18.5% (10/54 cases) for the 400/200 mg group. The ACR70 response rate was 3.7% (2/54 cases) for the placebo group, 3.6% (1/28 cases) for the 100 mg group, 9.3% (5/54 cases) for the 200 mg group, and 7.4% (4/54 cases) for the 400/200 mg group (Figure 3). The ACR20 response rate after week 24 was 35.2% (19/54 cases) for the placebo group, 39.3% (11/28 cases) for the 100 mg group, 53.7% (29/54 cases) for the 200 mg group, and 57.4% (31/54 cases) for the 400/200 mg group, and was thus significantly high in the 200 mg group and the 400/200 mg group compared with the placebo group. The ACR50 response rate was 16.7% (9/54 cases) for the placebo group, 17.9% (5/28 cases) for the 100 mg group, 25.9% (14/54 cases) for the 200 mg group, and 27.8% (15/54 cases) for the 400/200 mg group. The ACR70 response rate was 5.6% (3/54 cases) for the placebo group, 14.3% (4/28 cases) for the 100 mg group, 11.1% (6/54 cases) for the 200 mg group, and 13.0% (7/54 cases) for the 400/200 mg group (Figure 4). Data was complemented by non-responder imputation (NRI) as to cases found to have missing effectiveness data at each time of evaluation because of early discontinuation or other reasons.

### 3-4: Subgroup analysis based on CD16+ monocyte ratio

The ratio (percentage) of CD16+ monocytes to total monocytes in peripheral blood collected before administration (day 1) (baseline) was calculated in the same manner as the method described in the section 2-4 except that the fluorescently labeled anti-CD14 antibody was changed from Alexa Fluor 647-CD14 (BioLegend, Inc., 325612) to Brilliant Violet-CD14 (BioLegend, Inc., 325628). As in the section 3-3, effectiveness data was complemented by non-responder imputation (NRI) as to cases found to have missing effectiveness data at each time of evaluation because of early discontinuation or other reasons.

ACR response rates obtained by dividing the whole population into two at the median CD16+ monocyte ratio (10.35%) at the baseline will be given below. The ACR20 response rate after week 12 in a population having a CD16+ monocyte ratio less than the median value (< 10.35%) was 43.3% (13/30 cases) for the placebo group, 20.0% (2/10 cases) for the 100 mg group, 45.5% (10/22 cases) for the 200 mg group, and 27.3% (6/22 cases) for the 400 mg group. The ACR50 response rate was 13.3% (4/30 cases) for the placebo group, 10.0% (1/10 cases) for the 100 mg group, 22.7% (5/22 cases) for the 200 mg group, and 9.1% (2/22 cases) for the 400/200 mg group.

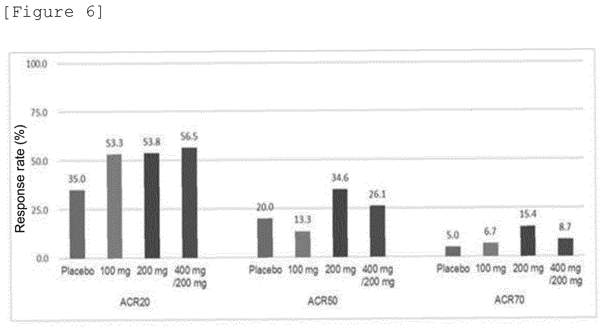

The ACR70 response rate was 3.3% (1/30 cases) for the placebo group, 0.0% (0/10 cases) for the 100 mg group, 4.5% (1/22 cases) for the 200 mg group, and 4.5% (1/22 cases) for the 400/200 mg group (Figure 5). On the other hand, the ACR20 response rate after week 12 in a population having a CD16+ monocyte ratio equal to or more than the median value (≥ 10.35%) was 35.0% (7/20 cases) for the placebo group, 53.3% (8/15 cases) for the 100 mg group, 53.8% (14/26 cases) for the 200 mg group, and 56.5% (13/23 cases) for the 400/200 mg group. The ACR50 response rate was 20.0% (4/20 cases) for the placebo group, 13.3% (2/15 cases) for the 100 mg group, 34.6% (9/26 cases) for the 200 mg group, and 26.1% (6/23 cases) for the 400/200 mg group. The ACR70 response rate was 5.0% (1/20 cases) for the placebo group, 6.7% (1/15 cases) for the 100 mg group, 15.4% (4/26 cases) for the 200 mg group, and 8.7% (2/23 cases) for the 400/200 mg group (Figure 6). This result suggested that the effectiveness of H3-2L4 is strongly detected in populations having a CD16+ monocyte ratio equal to or more than the median value.

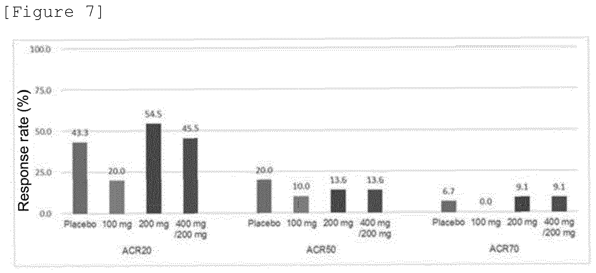

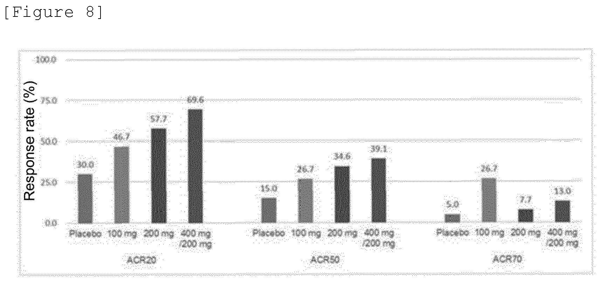

As for ACR20 response rates after week 24, the ACR20 response rate in a population having a CD16+ monocyte ratio less than the median value was 43.3% (13/30 cases) for the placebo group, 20.0% (2/10 cases) for the 100 mg group, 54.5% (12/22 cases) for the 200 mg group, and 45.5% (10/22 cases) for the 400/200 mg group. The ACR50 response rate was 20.0% (6/30 cases) for the placebo group, 10.0% (1/10 cases) for the 100 mg group, 13.6% (3/22 cases) for the 200 mg group, and 13.6% (3/22 cases) for the 400/200 mg group. ACR70 response rate was 6.7% (2/30 cases) for the placebo group, 0.0% (0/10 cases) for the 100 mg group, 9.1% (2/22 cases) for the 200 mg group, and 9.1% (2/22 cases) for the 400/200 mg group (Figure 7). On the other hand, the ACR20 response rate in a population having a CD16+ monocyte ratio equal to or more than the median value was 30.0% (6/20 cases) for the placebo group, 46.7% (7/15 cases) for the 100 mg group, 57.7% (15/26 cases) for the 200 mg group, and 69.6% (16/23 cases) for the 400/200 mg group. The ACR50 response rate was 15.0% (3/20 cases) for the placebo group, 26.7% (4/15 cases) for the 100 mg group, 34.6% (9/26 cases) for the 200 mg group, and 39.1% (9/23 cases) for the 400/200 mg group. The ACR70 response rate was 5.0% (1/20 cases) for the placebo group, 26.7% (4/15 cases) for the 100 mg group, 7.7% (2/26 cases) for the 200 mg group, and 13.0% (3/23 cases) for the 400/200 mg group (Figure 8). This result suggested that the effectiveness of H3-2L4 is detected with clearer dose responsiveness in populations having a CD16+ monocyte ratio equal to or more than the median value.

No bias occurred between the backgrounds of subjects in two groups when the whole population was divided into two at the median CD16+ monocyte ratio (10.35%) at the baseline. Thus, there was no possible confounder to be particular attended to in understanding patient stratification results based on the CD16+ monocyte ratio (Table 5).
[Table 5]

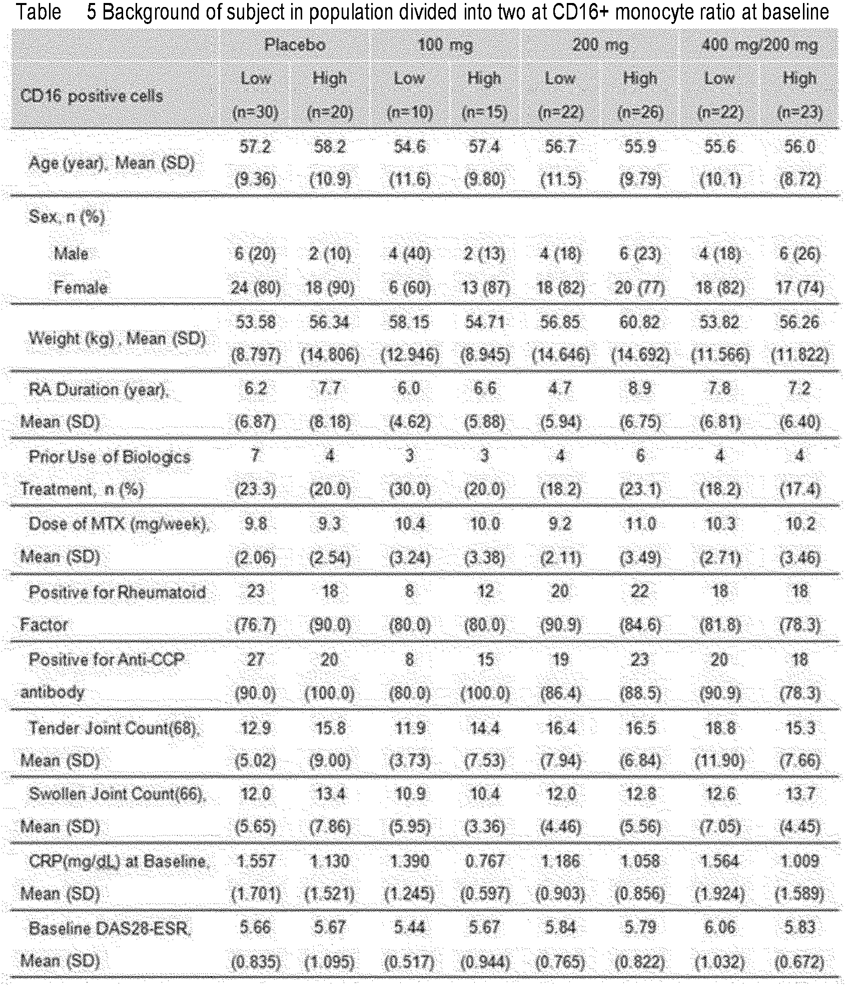

## Claims

1. A method for predicting a therapeutic effect of a drug that inhibits fractalkine (FKN)-CX3CR1 interaction in a rheumatoid arthritis subject, the method comprising
predicting the therapeutic effect of the drug in the subject on the basis of a measurement value of CD16+ monocytes in a biological sample obtained from the subject before the start of administration of the drug.

2. The method according to claim 1, wherein
the prediction comprises comparing the measurement value of CD16+ monocytes with a control.

3. The method according to claim 2, wherein
the measurement value of CD16+ monocytes equal to or higher than the control indicates that the subject is likely to respond to the drug.

4. The method according to any one of claims 1 to 3, wherein
the biological sample is blood or synovial fluid.

5. The method according to claim 4, wherein
the biological sample is blood.

6. The method according to any one of claims 1 to 5, wherein
the measurement value of CD16+ monocytes is a numerical value calculated from a ratio of the CD16+ monocytes to total monocytes.

7. The method according to any one of claims 1 to 6, wherein
the drug is an anti-human FKN antibody or an antibody binding fragment thereof.

8. The method according to claim 7, wherein
the anti-human FKN antibody is a fully human, humanized or chimeric antibody.

9. The method according to claim 7 or 8, wherein
the anti-human FKN antibody comprises
(a) CDR-H1 comprising the amino acid sequence of SEQ ID NO: 5 (NYYIH);
(b) CDR-H2 comprising the amino acid sequence of SEQ ID NO: 6 (WIYPGDGSPKFNERFKG);
(c) CDR-H3 comprising the amino acid sequence of SEQ ID NO: 7 (GPTDGDYFDY);
(d) CDR-L1 comprising the amino acid sequence of SEQ ID NO: 8 (RASGNIHNFLA);
(e) CDR-L2 comprising the amino acid sequence of SEQ ID NO: 9 (NEKTLAD); and
(f) CDR-L3 comprising the amino acid sequence of SEQ ID NO: 10 (QQFWSTPYT).

10. The method according to any one of claims 7 to 9, wherein
a heavy chain variable region of the anti-human FKN antibody comprises the amino acid sequence represented by SEQ ID NO: 3
(QVQLVQSGAEVKKPGASVKVSCKASGYTFTNYYIHWVKQAPGQGLEWIGWIYPGDG SPKFNERFKGRTTLTADKSTNTAYMLLSSLRSEDTAVYFCATGPTDGDYFDYWGQGT TVTVSS) and
a light chain variable region thereof comprises the amino acid sequence represented by SEQ ID NO: 4

(DIQMTQSPSSLSASVGDRVTITCRASGNIHNFLAWYQQKPGKAPKLLIYNEKTLAD

GVPSRFSGSGSGTDYTLTISSLQPEDFATYFCQQFWSTPYTFGGGTKVEIK).

11. The method according to any one of claims 7 to 10, wherein
the anti-human FKN antibody comprises a constant region of human IgG2 isotype, and
an Fc region of the constant region contains V234A and G237A mutations.

12. The method according to any one of claims 1 to 11, wherein
the rheumatoid arthritis subject is a rheumatoid arthritis patient with an inadequate response to conventional treatment.

13. A therapeutic agent for rheumatoid arthritis, comprising a drug that inhibits FKN-CX3CR1 interaction as an active ingredient, wherein
the therapeutic agent for rheumatoid arthritis is used in a method for treating rheumatoid arthritis, comprising the steps of:
identifying a subject predicted to be likely to respond to the drug on the basis of a measurement value of CD16+ monocytes in a biological sample obtained from a rheumatoid arthritis subject before the start of administration of the drug; and
administering a therapeutically effective amount of the drug to the identified subject.

14. The therapeutic agent for rheumatoid arthritis according to claim 13, wherein
the step of identifying a subject comprises identifying a subject in which the measurement value of CD16+ monocytes is equal to or higher than a control.

15. A therapeutic agent for rheumatoid arthritis, comprising a drug that inhibits FKN-CX3CR1 interaction as an active ingredient, wherein
the therapeutic agent for rheumatoid arthritis is used in a method for treating rheumatoid arthritis, comprising the step of
administering a therapeutically effective amount of the drug to a rheumatoid arthritis subject in which a measurement value of CD16+ monocytes in a biological sample obtained from the subject before the start of administration of the drug is equal to or higher than a control.

16. The therapeutic agent for rheumatoid arthritis according to any one of claims 13 to 15, wherein
the biological sample is blood or synovial fluid.

17. The therapeutic agent for rheumatoid arthritis according to claim 16, wherein
the biological sample is blood.

18. The therapeutic agent for rheumatoid arthritis according to any one of claims 13 to 17, wherein
the measurement value of CD16+ monocytes is a numerical value calculated from a ratio of the CD16+ monocytes to total monocytes.

19. The therapeutic agent for rheumatoid arthritis according to any one of claims 13 to 18, wherein
the drug is an anti-human FKN antibody or an antibody binding fragment thereof.

20. The therapeutic agent for rheumatoid arthritis according to claim 19, wherein
the anti-human FKN antibody is a fully human, humanized, or chimeric antibody.

21. The therapeutic agent for rheumatoid arthritis according to claim 19 or 20, wherein
the anti-human FKN antibody comprises
(a) CDR-H1 comprising the amino acid sequence of SEQ ID NO: 5 (NYYIH);
(b) CDR-H2 comprising the amino acid sequence of SEQ ID NO: 6 (WIYPGDGSPKFNERFKG);
(c) CDR-H3 comprising the amino acid sequence of SEQ ID NO: 7 (GPTDGDYFDY);
(d) CDR-L1 comprising the amino acid sequence of SEQ ID NO: 8 (RASGNIHNFLA);
(e) CDR-L2 comprising the amino acid sequence of SEQ ID NO: 9 (NEKTLAD); and
(f) CDR-L3 comprising the amino acid sequence of SEQ ID NO: 10 (QQFWSTPYT).

22. The therapeutic agent for rheumatoid arthritis according to any one of claims 19 to 21, wherein
a heavy chain variable region of the anti-human FKN antibody comprises the amino acid sequence represented by SEQ ID NO: 3
(QVQLVQSGAEVKKPGASVKVSCKASGYTFTNYYIHWVKQAPGQGLEWIGWIYPGDG SPKFNERFKGRTTLTADKSTNTAYMLLSSLRSEDTAVYFCATGPTDGDYFDYWGQGT TVTVSS) and
a light chain variable region thereof comprises the amino acid sequence represented by SEQ ID NO: 4

(DIQMTQSPSSLSASVGDRVTITCRASGNIHNFLAWYQQKPGKAPKLLIYNEKTLAD

GVPSRFSGSGSGTDYTLTISSLQPEDFATYFCQQFWSTPYTFGGGTKVEIK).

23. The therapeutic agent for rheumatoid arthritis according to any one of claims 19 to 22, wherein
the anti-human FKN antibody comprises a constant region of human IgG2 isotype, and
an Fc region of the constant region contains V234A and G237A mutations.

24. The therapeutic agent for rheumatoid arthritis according to any one of claims 13 to 23, wherein
the rheumatoid arthritis subject is a rheumatoid arthritis patient with an inadequate response to conventional treatment.

25. A method for treating rheumatoid arthritis, comprising the steps of:
identifying a subject predicted to be likely to respond to a drug that inhibits FKN-CX3CR1 interaction on the basis of a measurement value of CD16+ monocytes in a biological sample obtained from a rheumatoid arthritis subject; and
administering a therapeutically effective amount of the drug to the identified subject.

26. The method for treating rheumatoid arthritis according to claim 25, wherein
the step of identifying a subject comprises identifying a subject who is equal to or higher than a control.

27. A method for treating rheumatoid arthritis, comprising the step of
administering a therapeutically effective amount of a drug that inhibits FKN-CX3CR1 interaction to a rheumatoid arthritis subject in which a measurement value of CD16+ monocytes in a biological sample obtained from the subject before the start of administration of the drug is equal to or higher than a control.

28. The method for treating rheumatoid arthritis according to any one of claims 25 to 27, wherein
the biological sample is blood or synovial fluid.

29. The method for treating rheumatoid arthritis according to claim 28, wherein
the biological sample is blood.

30. The method for treating rheumatoid arthritis according to any one of claims 25 to 29, wherein
the measurement value of CD16+ monocytes is a numerical value calculated from a ratio of the CD16+ monocytes to total monocytes.

31. The method for treating rheumatoid arthritis according to any one of claims 25 to 30, wherein
the drug is an anti-human FKN antibody or an antibody binding fragment thereof.

32. The method for treating rheumatoid arthritis according to claim 31, wherein
the anti-human FKN antibody is a fully human, humanized or chimeric antibody.

33. The method for treating rheumatoid arthritis according to claim 31 or 32, wherein
the anti-human FKN antibody comprises
(a) CDR-H1 comprising the amino acid sequence of SEQ ID NO: 5 (NYYIH);
(b) CDR-H2 comprising the amino acid sequence of SEQ ID NO: 6 (WIYPGDGSPKFNERFKG);
(c) CDR-H3 comprising the amino acid sequence of SEQ ID NO: 7 (GPTDGDYFDY);
(d) CDR-L1 comprising the amino acid sequence of SEQ ID NO: 8 (RASGNIHNFLA);
(e) CDR-L2 comprising the amino acid sequence of SEQ ID NO: 9 (NEKTLAD); and
(f) CDR-L3 comprising the amino acid sequence of SEQ ID NO: 10 (QQFWSTPYT).

34. The method for treating rheumatoid arthritis according to any one of claims 31 to 33, wherein
a heavy chain variable region of the anti-human FKN antibody comprises the amino acid sequence represented by SEQ ID NO: 3
(QVQLVQSGAEVKKPGASVKVSCKASGYTFTNYYIHWVKQAPGQGLEWIGWIYPGDG SPKFNERFKGRTTLTADKSTNTAYMLLSSLRSEDTAVYFCATGPTDGDYFDYWGQGT TVTVSS) and
a light chain variable region thereof comprises the amino acid sequence represented by SEQ ID NO: 4

(DIQMTQSPSSLSASVGDRVTITCRASGNIHNFLAWYQQKPGKAPKLLIYNEKTLAD

GVPSRFSGSGSGTDYTLTISSLQPEDFATYFCQQFWSTPYTFGGGTKVEIK).

35. The method for treating rheumatoid arthritis according to any one of claims 31 to 34, wherein
the anti-human FKN antibody comprises a constant region of human IgG2 isotype, and
an Fc region of the constant region contains V234A and G237A mutations.

36. The method for treating rheumatoid arthritis according to any one of claims 25 to 35, wherein
the rheumatoid arthritis subject is a rheumatoid arthritis patient with an inadequate response to conventional treatment.


SEQUENCE LISTING

<110>  EISAI R&D MANAGEMENT CO., LTD.

<120>  Biomarker for Treatment of Rheumatoid Arthritis

<130>  ESAP1902F

<150>  JP2019-081452
<151>  2019.04.23

<160>  16

<170>  PatentIn version 3.5

<210>  1
<211>  445
<212>  PRT
<213>  Artificial Sequence

<220>
<223>  Synthetic polypeptide

<400>  1

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asn Tyr
            20                  25                  30

Tyr Ile His Trp Val Lys Gln Ala Pro Gly Gln Gly Leu Glu Trp Ile
        35                  40                  45

Gly Trp Ile Tyr Pro Gly Asp Gly Ser Pro Lys Phe Asn Glu Arg Phe
    50                  55                  60

Lys Gly Arg Thr Thr Leu Thr Ala Asp Lys Ser Thr Asn Thr Ala Tyr
65                  70                  75                  80

Met Leu Leu Ser Ser Leu Arg Ser Glu Asp Thr Ala Val Tyr Phe Cys
                85                  90                  95

Ala Thr Gly Pro Thr Asp Gly Asp Tyr Phe Asp Tyr Trp Gly Gln Gly
            100                 105                 110

Thr Thr Val Thr Val Ser Ser Ala Ser Thr Lys Gly Pro Ser Val Phe
        115                 120                 125

Pro Leu Ala Pro Cys Ser Arg Ser Thr Ser Glu Ser Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Asn Phe Gly Thr Gln Thr Tyr Thr Cys Asn Val Asp His Lys Pro
        195                 200                 205

Ser Asn Thr Lys Val Asp Lys Thr Val Glu Arg Lys Cys Cys Val Glu
    210                 215                 220

Cys Pro Pro Cys Pro Ala Pro Pro Ala Ala Ala Pro Ser Val Phe Leu
225                 230                 235                 240

Phe Pro Pro Lys Pro Lys Asp Thr Leu Met Ile Ser Arg Thr Pro Glu
                245                 250                 255

Val Thr Cys Val Val Val Asp Val Ser His Glu Asp Pro Glu Val Gln
            260                 265                 270

Phe Asn Trp Tyr Val Asp Gly Val Glu Val His Asn Ala Lys Thr Lys
        275                 280                 285

Pro Arg Glu Glu Gln Phe Asn Ser Thr Phe Arg Val Val Ser Val Leu
    290                 295                 300

Thr Val Val His Gln Asp Trp Leu Asn Gly Lys Glu Tyr Lys Cys Lys
305                 310                 315                 320

Val Ser Asn Lys Gly Leu Pro Ala Pro Ile Glu Lys Thr Ile Ser Lys
                325                 330                 335

Thr Lys Gly Gln Pro Arg Glu Pro Gln Val Tyr Thr Leu Pro Pro Ser
            340                 345                 350

Arg Glu Glu Met Thr Lys Asn Gln Val Ser Leu Thr Cys Leu Val Lys
        355                 360                 365

Gly Phe Tyr Pro Ser Asp Ile Ala Val Glu Trp Glu Ser Asn Gly Gln
    370                 375                 380

Pro Glu Asn Asn Tyr Lys Thr Thr Pro Pro Met Leu Asp Ser Asp Gly
385                 390                 395                 400

Ser Phe Phe Leu Tyr Ser Lys Leu Thr Val Asp Lys Ser Arg Trp Gln

                405                 410                 415

Gln Gly Asn Val Phe Ser Cys Ser Val Met His Glu Ala Leu His Asn
            420                 425                 430

His Tyr Thr Gln Lys Ser Leu Ser Leu Ser Pro Gly Lys
        435                 440                 445

<210> 2
<211> 214
<212> PRT
<213> Artificial Sequence

<220>
<223> Synthetic polypeptide

<400> 2

Asp Ile Gln Met Thr Gln Ser Pro Ser Ser Leu Ser Ala Ser Val Gly
1               5                   10                  15

Asp Arg Val Thr Ile Thr Cys Arg Ala Ser Gly Asn Ile His Asn Phe
            20                  25                  30

Leu Ala Trp Tyr Gln Gln Lys Pro Gly Lys Ala Pro Lys Leu Leu Ile
        35                  40                  45

Tyr Asn Glu Lys Thr Leu Ala Asp Gly Val Pro Ser Arg Phe Ser Gly
    50                  55                  60

Ser Gly Ser Gly Thr Asp Tyr Thr Leu Thr Ile Ser Ser Leu Gln Pro
65                  70                  75                  80

Glu Asp Phe Ala Thr Tyr Phe Cys Gln Gln Phe Trp Ser Thr Pro Tyr
                85                  90                  95

Thr Phe Gly Gly Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  3
<211>  119
<212>  PRT
<213>  Artificial Sequence

<220>
<223>  Synthetic polypeptide

<400>  3

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asn Tyr
            20                  25                  30

Tyr Ile His Trp Val Lys Gln Ala Pro Gly Gln Gly Leu Glu Trp Ile
        35                  40                  45

Gly Trp Ile Tyr Pro Gly Asp Gly Ser Pro Lys Phe Asn Glu Arg Phe
    50                  55                  60

Lys Gly Arg Thr Thr Leu Thr Ala Asp Lys Ser Thr Asn Thr Ala Tyr
65                  70                  75                  80

Met Leu Leu Ser Ser Leu Arg Ser Glu Asp Thr Ala Val Tyr Phe Cys
                85                  90                  95

Ala Thr Gly Pro Thr Asp Gly Asp Tyr Phe Asp Tyr Trp Gly Gln Gly
            100                 105                 110

Thr Thr Val Thr Val Ser Ser
        115

<210>  4
<211>  107
<212>  PRT
<213>  Artificial Sequence

<220>
<223>  Synthetic polypeptide

<400>  4

Asp Ile Gln Met Thr Gln Ser Pro Ser Ser Leu Ser Ala Ser Val Gly
1               5                   10                  15

Asp Arg Val Thr Ile Thr Cys Arg Ala Ser Gly Asn Ile His Asn Phe
            20                  25                  30

Leu Ala Trp Tyr Gln Gln Lys Pro Gly Lys Ala Pro Lys Leu Leu Ile
        35                  40                  45

Tyr Asn Glu Lys Thr Leu Ala Asp Gly Val Pro Ser Arg Phe Ser Gly
    50                  55                  60

Ser Gly Ser Gly Thr Asp Tyr Thr Leu Thr Ile Ser Ser Leu Gln Pro
65                  70                  75                  80

Glu Asp Phe Ala Thr Tyr Phe Cys Gln Gln Phe Trp Ser Thr Pro Tyr
                85                  90                  95

Thr Phe Gly Gly Gly Thr Lys Val Glu Ile Lys
            100                 105

<210>  5
<211>  5
<212>  PRT
<213>  Artificial Sequence

<220>
<223>  Synthetic polypeptide

<400>  5

Asn Tyr Tyr Ile His
1               5

<210>  6
<211>  17
<212>  PRT
<213>  Artificial Sequence

<220>
<223>  Synthetic polypeptide

<400>  6

Trp Ile Tyr Pro Gly Asp Gly Ser Pro Lys Phe Asn Glu Arg Phe Lys
1               5                   10                  15

Gly

<210>  7
<211>  10
<212>  PRT
<213>  Artificial Sequence

<220>
<223>  Synthetic polypeptide

<400>  7

Gly Pro Thr Asp Gly Asp Tyr Phe Asp Tyr
1               5                   10


<210>  8
<211>  11
<212>  PRT
<213>  Artificial Sequence

<220>
<223>  Synthetic polypeptide

<400>  8

Arg Ala Ser Gly Asn Ile His Asn Phe Leu Ala
1               5                   10


<210>  9
<211>  7
<212>  PRT
<213>  Artificial Sequence

<220>
<223>  Synthetic polypeptide

<400>  9

Asn Glu Lys Thr Leu Ala Asp
1               5


<210>  10
<211>  9
<212>  PRT
<213>  Artificial Sequence

<220>
<223>  Synthetic polypeptide

<400>  10

Gln Gln Phe Trp Ser Thr Pro Tyr Thr
1               5


<210>  11
<211>  119
<212>  PRT
<213>  Artificial Sequence

<220>
<223>  Synthetic polypeptide

<400>  11

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asn Tyr
            20                  25                  30

Tyr Ile His Trp Val Lys Gln Ala Pro Gly Gln Gly Leu Glu Trp Ile
        35                  40                  45

Gly Trp Ile Tyr Pro Gly Asp Gly Ser Pro Lys Phe Asn Glu Arg Phe
    50                  55                  60

Lys Gly Arg Thr Thr Leu Thr Ala Asp Lys Ser Thr Asn Thr Ala Tyr
65                  70                  75                  80

Met Leu Leu Ser Ser Leu Arg Ser Asp Asp Thr Ala Val Tyr Phe Cys
                85                  90                  95

Ala Thr Gly Pro Thr Asp Gly Asp Tyr Phe Asp Tyr Trp Gly Gln Gly
            100                 105                 110

Thr Thr Val Thr Val Ser Ser
        115


<210>  12
<211>  119
<212>  PRT
<213>  Artificial Sequence

<220>
<223>  Synthetic polypeptide

<400>  12

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asn Tyr
            20                  25                  30

Tyr Ile His Trp Val Arg Gln Ala Pro Gly Gln Gly Leu Glu Trp Ile
        35                  40                  45

Gly Trp Ile Tyr Pro Gly Asp Gly Ser Pro Lys Phe Asn Glu Arg Phe
    50                  55                  60

Lys Gly Arg Thr Thr Leu Thr Arg Asp Lys Ser Thr Asn Thr Ala Tyr
65                  70                  75                  80

Met Glu Leu Ser Ser Leu Arg Ser Asp Asp Thr Ala Val Tyr Phe Cys
                85                  90                  95

Ala Thr Gly Pro Thr Asp Gly Asp Tyr Phe Asp Tyr Trp Gly Gln Gly
            100                 105                 110

Thr Thr Val Thr Val Ser Ser
        115

<210>  13
<211>  119
<212>  PRT
<213>  Artificial Sequence

<220>
<223>  Synthetic polypeptide

<400>  13

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asn Tyr
            20                  25                  30

Tyr Ile His Trp Val Arg Gln Ala Pro Gly Gln Gly Leu Glu Trp Ile
        35                  40                  45

Gly Trp Ile Tyr Pro Gly Asp Gly Ser Pro Lys Phe Asn Glu Arg Phe
    50                  55                  60

Lys Gly Arg Thr Thr Met Thr Ala Asp Thr Ser Thr Ser Thr Ala Tyr
65                  70                  75                  80

Met Glu Leu Ser Ser Leu Arg Ser Glu Asp Thr Ala Val Tyr Phe Cys
                85                  90                  95

Ala Arg Gly Pro Thr Asp Gly Asp Tyr Phe Asp Tyr Trp Gly Gln Gly
            100                 105                 110

Thr Thr Val Thr Val Ser Ser
        115

<210>  14
<211>  119
<212>  PRT
<213>  Artificial Sequence

<220>
<223>  Synthetic polypeptide

<400>  14

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asn Tyr
            20                  25                  30

Tyr Ile His Trp Val Arg Gln Ala Pro Gly Gln Gly Leu Glu Trp Ile
        35                  40                  45

Gly Trp Ile Tyr Pro Gly Asp Gly Ser Pro Lys Phe Asn Glu Arg Phe
    50                  55                  60

Lys Gly Arg Thr Thr Leu Thr Ala Asp Lys Ser Thr Ser Thr Ala Tyr
65                  70                  75                  80

Met Glu Leu Ser Ser Leu Arg Ser Glu Asp Thr Ala Val Tyr Phe Cys
                85                  90                  95

Ala Arg Gly Pro Thr Asp Gly Asp Tyr Phe Asp Tyr Trp Gly Gln Gly
            100                 105                 110

Thr Thr Val Thr Val Ser Ser
        115


<210>  15
<211>  107
<212>  PRT
<213>  Artificial Sequence

<220>
<223>  Synthetic polypeptide

<400>  15

Asp Ile Gln Met Thr Gln Ser Pro Ser Ser Leu Ser Ala Ser Val Gly
1               5                   10                  15

Asp Arg Val Thr Ile Thr Cys Arg Ala Ser Gly Asn Ile His Asn Phe
            20                  25                  30

Leu Ala Trp Tyr Gln Gln Lys Pro Gly Lys Ala Pro Lys Phe Leu Val
        35                  40                  45

Tyr Asn Glu Lys Thr Leu Ala Asp Gly Val Pro Ser Arg Phe Ser Gly
    50                  55                  60

Ser Gly Ser Gly Thr Gln Tyr Thr Leu Thr Ile Ser Ser Leu Gln Pro
65                  70                  75                  80

Glu Asp Phe Ala Thr Tyr Phe Cys Gln Gln Phe Trp Ser Thr Pro Tyr
                85                  90                  95

Thr Phe Gly Gly Gly Thr Lys Val Glu Ile Lys
            100                 105

<210>  16
<211>  107
<212>  PRT
<213>  Artificial Sequence

<220>
<223>  Synthetic polypeptide

<400>  16

Asp Ile Gln Met Thr Gln Ser Pro Ser Ser Leu Ser Ala Ser Val Gly
1               5                   10                  15

Asp Arg Val Thr Ile Thr Cys Arg Ala Ser Gly Asn Ile His Asn Phe
            20                  25                  30

Leu Ala Trp Tyr Gln Gln Lys Pro Gly Lys Ala Pro Lys Leu Leu Ile
        35                  40                  45

Tyr Asn Glu Lys Thr Leu Ala Asp Gly Val Pro Ser Arg Phe Ser Gly
    50                  55                  60

Ser Gly Ser Gly Thr Gln Tyr Thr Leu Thr Ile Ser Ser Leu Gln Pro
65                  70                  75                  80

Glu Asp Phe Ala Thr Tyr Phe Cys Gln Gln Phe Trp Ser Thr Pro Tyr
                85                  90                  95

Thr Phe Gly Gly Gly Thr Lys Val Glu Ile Lys
            100                 105